# DEMANDE DE BREVET EUROPEEN

(11) **EP 2 199 413 A1**
(43) Date de publication de la demande: **23.06.2010**
(21) Numéro de dépôt: 08291064.7
(22) Date de dépôt: 13.11.2008
(51) Int. Cl.: C12Q 1/68

(54) **Procédé de détection de la maladie parodontale**

(71) Demandeur: Cohen-Loro, Yves, 92400 Courbevoie (FR)
(72) Inventeur: Cohen-Loro, Yves, 92400 Courbevoie (FR)
(74) Mandataire: Ulmann, Catherine Claire

(57) **Abrégé**

L'invention concerne un procédé de détection de la maladie parodontale utilisant des tests bactériens et génétiques.

## Description

### PREAMBULE

L'invention concerne un procédé de détection de la maladie parodontale utilisant des tests bactériens et génétiques.

### PREAMBULE

Le terme générique de «parodontopathie» regroupe un ensemble d'affections très diverses et variées qui, en France, encore aujourd'hui, concernent 90 % de la population, et responsables de la perte de 30 à 40% des dents.

Même si
97 % des patients présentent une inflammation superficielle et une perte d'attache de 1 mm,
40 % de la population, une perte d'attache de 3mm et 15 %, de plus de 3mm.

Sans traitement, ces patients risquent de façon majeure, dans les années à venir, des désordres variables jusqu' au déficit dentaire (117).

Usuellement, une irritation de nature inflammatoire de la gencive marginale définit «la gingivite» et l'altération des tissus de soutien de la dent avec perte d'ancrage, «la parodontite».

Les connaissances étiopathogéniques actuelles de la maladie parodontale ne nous permettent pas de présumer si une gingivite entraînera une parodontite ou si elle se stabilisera.

Cette «présentation» de la maladie parodontale toute vulgarisée qu'elle soit, reste la base de sa définition, dans toute sa convention.

Dès 1999, ARMITAGE présente une classification étiologique et clinique, très complète, reconnue universellement à ce jour. (3)

Un aperçu actualisé des données récentes confirme sans équivoque l'étiologie locale bactérienne des maladies parodontales et insiste sur la grande variété de la flore (plus de 500 espèces recensées), ainsi que la complexité et diversité des réponses de l'organisme face à l'activité des souches pathogènes.

Ces réponses biologiques de l'hôte, expression d'une capacité immunitaire et génétique de défense, contribuent à la réactivité de cet écosystème - agents locaux/ hôte.

Encore aujourd'hui, l'approche épidémiologique des paramètres endogènes et exogènes- influençant la susceptibilité de ce complexe est loin d'être exhaustive.

Tous les auteurs conviennent de l'extrême difficulté à identifier la nature et quantifier la part relative des différents facteurs modulant la *pathogénicité -* voire la *virulence-* des souches locales et de ladite réponse de l'hôte.

Le choix de ces deux termes fait déjà résonance et engage le débat!

L'analyse des plus récents articles, toutes disciplines confondues, nous invite à une réflexion et,peut-être, à une proposition d'une définition autrement ciblée de la maladie parodontale et d'en décanter un caractère fonctionnel, leitmotiv retenu dans de nombreux articles.

Alors, il se pourrait que la définition bactériologique de la maladie parodontale ne présente plus seulement un caractère cognitif mais aussi une tendance diagnostique, sous réserve d'une anamnèse épidémiologique.

Rationaliser cette anamnèse et séquencer l'approche diagnostique présentent toujours un défi impressionnant, mais il semblerait que, au terme de cet effort, on puisse espérer des conséquences très positives en matière de prévention et de soutien parodontal.

Une revue de la littérature récente laisse à penser que la Parodontie risque non seulement de connaître un essor tout particulier, mais encore de devenir une discipline tremplin par les perspectives qu'elle offre.

Le fluide gingival n'a pas encore livré tous ses secrets ni les perspectives de traçabilité en matière de prévention et diagnostic, y compris en médecine générale et thérapeutique de pointe.

### La «troisième voie»

A ce jour, deux courants de pensée cohabitent en matière de stratégie parodontale:

Le premier qui considère que la maladie parodontale est provoquée essentiellement par l'activité microbienne. La flore, cause locale majeure, étant alors reconnue comme référant. (**voie 1**)

Autour de ce facteur, gravitent tout un ensemble de «co-effectants».

Alors associés, ils composent un écosystème caractérisant un état aux critères propices à l'expression plus ou moins avérée de la maladie.

Nous pouvons lire tous les développements à propos du biofilm, des associations bactériennes, de la discussion concernant la plus ou moins relative non- spécificité bactérienne, les synergies, les antagonismes bactériens, l'enzymologie associée aux caractéristiques de leur catabolisme, les résistances, les controverses déclenchées par la pertinence contestée des traitements antibiotiques locaux.

Le deuxième, qui désigne un autre axe de réflexion fondé sur le constat que la réponse de l'hôte est, sinon spécifique, au moins singulier. (**voie 2**)

À une pathogénie complexe, l'organisme déclenche des moyens de sauvegarde et de défense qui se manifestent, par définition, pour nombre d'entre eux, au titre de signaux électifs ciblés.

Ainsi, pouvons-nous citer la salive et ses composants, les processus inflammatoires, la voie du complément, la réponse immunitaire non spécifique et spécifique, la génétique et la cascade réactive de l'INF α et les interleukines, la génétique et la transmission mendélienne de certaines formes de parodontites.

N'oublions pas les dérèglements liés à la consommation tabagique, et plus récemment une approche encore plus implicite évoquant le stress oxydatif et la fabrication de radicaux libres, le principe biologique du vieillissement cellulaire et de l'apoptose, et, encore, les interférences avec des maladies générales, notamment dégénératives et tumorales...

### INTRODUCTION

Les maladies parodontales sont reconnues de nature inflammatoire d'étiologie microbienne:

Elles résultent d'interactions entre les systèmes immunitaires et inflammatoires et les bactéries des biofilms. Elles concernent un ensemble de tissus mous (épithélium et conjonctif gingival, ligament alvéolo- dentaire) et de tissus minéralisés (os, cément).

Leur progression discontinue alterne des épisodes actifs et des périodes de latence.

La très large échelle de sévérité de lésions affectant des sites différents chez un même patient, confirme que les informations diagnostiques se doivent d'aller bien au- delà d'un répertoire habituel distribuant la maladie en classes et en sous- classes cliniques (Mc CULLOUGH, 1994).

Nos connaissances actuelles en Microbiologie, adaptées à l'environnement parodontal (biofilm, composition évolutive de la flore pathogène,...) relativisent le caractère non spécifique de la flore bactérienne.

Des publications récentes font remarquer que" la définition bactériologique de la maladie parodontale est cognitive et non diagnostique." et aussi, " nous ne disposons pas encore à ce jour de classification fonctionnelle des maladies parodontales"

La capacité des bactéries à former des biofilms constitue un caractère potentialisant leur pathogénicité, voire leur virulence (aspect qualitatif et/ ou quantitatif). Cet écosystème définit le complexe étiologique capital de la maladie parodontale.

Les mécanismes de défense de l'hôte servent alors de relais précieux aux outils thérapeutiques, et aux moyens d'investigation à valeur diagnostique, pronostique et préventive.

Les tests biologiques préalables- microbiologique et génétique- prennent tout leur sens.

Ils renforcent l'acuité des traitements non chirurgicaux d'assainissement parodontal.

Les connaissances de l'histoétiopathogénie et des changements de comportements biochimiques et cellulaires associés à la mise en place des lésions parodontales, notamment agressives, guident les laboratoires et les praticiens dans l'élaboration et la mise en oeuvre clinique- actuelles et à venir- de ces examens complémentaires diagnostics.

Le traitement des affections parodontales d'origine bactérienne s'est beaucoup affiné depuis trente ans jusqu'à, de nos jours, aboutir au concept de traitement non chirurgical.

Ce concept propose un protocole efficace puisqu' il remplace, dans presque tous les cas, les techniques chirurgicales d'assainissement.

D'une part, non invasif, il répond à un rythme physiologique de réparation de l'attache épithélio-conjonctive tout en ciblant une étiologie multi-microbienne.

D'autre part, sa mise en place mobilise le patient dès la phase préparatoire au traitement.

Il dispose de plus de temps pour intégrer la nécessité de sa coopération active au bon déroulement de la thérapeutique et de la prévention.

Il est important d'actualiser les interrelations entre les différentes spécialités médicales, notamment la génétique médicale, qui, par l'émergence d'une connaissance nouvelle concernant les gènes humains, a redéfini sa contribution dans l'analyse, le diagnostic, le traitement et la prévention de la maladie en général et de certaines formes parodontales en particulier.

On peut sans difficulté imaginer que les thérapeutes de ces prochaines années auront besoin d'une connaissance en génétique aussi étendue que possible.

En ce qui nous concerne, une pratique de la médecine buccale reposant sur l'intuition ou des expériences anecdotiques, bien qu'utile à un moment donné de son histoire, est aujourd'hui obsolète, parce que l'approche plus rationnelle des causes premières de la maladie nous impose la rigueur de la méthode scientifique.

Partie intégrante de la pratique médicale, la génétique aujourd'hui n'est plus strictement cette science fondamentale de ces vingt ou trente dernières années mais nous apporte, par ses progrès rapides, un savoir applicable en clinique. Les causes génétiques de la maladie apparaissent fréquemment dans nos sociétés modernes. Il est possible désormais de mettre à profit un savoir-faire technologique pour éviter ou prévenir certaines formes de la maladie.

Dans un contexte où l'accent est mis sur les symptômes et le traitement plutôt que l'étiologie et la pathogénèse, les principes de la génétique médicale prennent tout leur poids.

Elle ouvre des perspectives nouvelles de la compréhension des maladies.

Il est du domaine d'investigation des scientifiques de localiser, identifier les gènes, caractériser leur mutation, étudier la nature de leur produit. Ce, pour accéder à une meilleure compréhension d'un grand nombre de maladies dont la nature est, soit peu comprise, soit inconnue.

Une caractéristique majeure de la cavité buccale est sa septicité.

De ce fait, est-il réaliste de faire superposer le modèle pathologique buccal à celui des maladies infectieuses spécifiques qui concernent des appareils fonctionnant fort heureusement en asepsie?

La répartition des bactéries pathogènes en complexes (93) et la distribution sélective des différentes familles microbiennes identifiées selon les différentes classes de maladies parodontales (91) nous interpellent et invitent à une " lecture analytique transverse" des données acquises pour interpréter peut-être autrement les mécanismes des parodontopathies.

Ainsi, nous nous proposons ici, sous forme d'éléments de réflexion, de suivre un autre axe de classification de la maladie parodontale - étiologique et fonctionnelle. (**voie 3**) Cette classification, en complément de la nomenclature existante, serait applicable en clinique grâce à une fiche clinique synoptique simplifiée de diagnostic.

Chacun pourra y lire une orientation possible du traitement pour chaque forme rencontrée, en s'appuyant aussi sur un moyen simple et reproductible- un indice clinique recomposé, intégré à l'examen bio-clinique, pour cibler les patients à risque.

### 1. LA VOIE 1

### 1.1 Le biofilm

Les bactéries planctoniques, non fixées nageant librement dans un liquide, acquièrent une pathogénicité accrue dès lors qu'elles s'organisent en biofilm, dont la plaque dentaire est un exemple original.

C'est le cas de l'immense majorité des bactéries (90% estimés), qui acquièrent une densité 1000 fois supérieure.

De ce fait, les études doivent porter essentiellement sur les modalités de formation et le développement des biofilms multi-espèces fixés sur des surfaces.

### 1-1-1- Définitions

Les dents, dans tout l'organisme humain, représentent la seule surface solide permanente exposée à la colonisation bactérienne. Ces surfaces, dans l'environnement buccal, sont rapidement recouvertes de polymères protéiques salivaires adsorbés, *pellicule acquise salivaire*, sans bactérie.

La *plaque dentaire* s'en distingue par l'ensemble de populations microbiennes intriquées dans une matrice où elles adhèrent aux surfaces et interfaces. (36) Ce *biofilm bactérien* est classiquement défini comme une communauté bactérienne sessile caractérisée par des éléments irréversiblement fixés à un substrat et/ ou une interface, enfouis dans une matrice exopolysaccharidique qu'ils ont produite.

### 1.1.1.1 Notions biophysiques du biofilm

La formation de cette pellicule répond au phénomène de bioadhérence à toutes les interfaces entre les biofilms et les surfaces solides.

La grande variété de protéines salivaires à effet mouillant s'adsorbe sélectivement à toute surface solide, naturelle ou artificielle, exposée en bouche.

Des forces physiques, réduisant l'énergie libre aux interfaces, sont régulées par des forces électrostatiques, combinées à d'autres formes de participation, comme des modifications structurelles, favorisant l'adhésion de façon entropique.

Les interactions hydrophobes, sources majeures de force d'adsorption, seraient le résultat thermodynamique d'un déplacement d'eau structurée sur une surface par une protéine d'adsorption.

Ces protéines de l'environnement aqueux buccal marqueraient ainsi une prédisposition forte pour l'adsorption à la fois sur des surfaces polymériques hydrophobes céramiques et métalliques hydrophiles. (72)

### 1.1.1.2 Agrégats et conglomérats. Incidences biologiques

Des études récentes laissent à penser que des agrégats plutôt que des protéines simples contribueraient à former la pellicule initiale (globules d'allure micellaire parotidienne).

Cette pellicule subit ensuite un remaniement continuel par modification chimique et enzymatique des protéines entraînant adsorption et dés-adsorption.

Dès lors que des éléments figurés s'intègrent dans cette trame agrégée, on pourrait parler de conglomérat, terme de Géologie sédimentaire, qui sous-entend un comportement dissocié mais de proximité entre la trame "contenant" et les éléments "contenus".

Ces remaniements favorisent l'intégration des divers bio-composants du fluide d'origine salivaire, du fluide gingival, du sang, des bactéries, des muqueuses, même de l'alimentation, des virus et mycoplasmes.

Protéines, glycoprotéines, enzymes, mucines ou leurs dérivés respectifs en sont les composants majeurs.

Parmi les constituants spécifiques ont été identifiés les protéines riches en proline, histatines, statherine, cystatine, alpha-amylase, albumine, lysozyme, lactoferrine, anhydrases carboniques, immunoglobulines, mucines, agglutinine salivaire et des composants bactériens, les glycosyltransférases. (4, 36, 110)

### 1.1.1.3 Critères influençant le milieu

Moins de 3 minutes sont nécessaires pour l'adsorption de protéines simples détectables inhibant l'activité enzymatique au niveau du biofilm. La pellicule intervient au cours de tout évènement se produisant à l'interface de la surface dentaire.

De ce fait, elle joue un rôle majeur physiologique et physiopathologique.

Au-delà de ses rôles physico-chimiques, tels que effet de barrière mécanique et tampon vis-à-vis des agents de déminéralisation, c'est aussi un réservoir d'électrolytes de reminéralisation et un moyen antimicrobien par l'activité du lysozyme et des IgAs.

Le lysozyme, bactériolytique majeur, lié à la pellicule et l'alpha-amylase, enzyme structurelle, reste actif au sein de la pellicule même plusieurs minutes après sa formation, de même pour la glycosyltransférase, facteur de virulence bactérien.

Contrairement à la plaque dentaire, la pellicule constitue un lubrifiant résistant à son élimination par l'effet mécanique des techniques d'hygiène. Ce caractère lubrifiant extrêmement tenace confère une propriété de contrôle et protection de l'équilibre minéral à la surface amélaire, propriété partiellement amoindrie par le développement ultérieur de la plaque dentaire. (36, 72, 110)

### 1.1.1.3.1. Le biofilm et les souches pathogènes

Plus le biofilm dentaire est jeune, plus les pourcentages de bactéries vivantes / viables qu'il contient sont faibles. (110)

La capacité biophysique d'adhérence des populations microbiennes leur permet de coloniser le volume sous-gingival et d'assurer leur survie dans un milieu soumis en permanence aux flux de la salive et du fluide sulculaire.

### 1-1-1-3-2- Adhérence inter bactérienne et bactéries-interface

Des protéines salivaires spécifiques immobilisées dans la pellicule se lient sélectivement à des bactéries planctoniques des fluides buccaux.

Les bactéries gram+ saccharolytiques interagissent avec l'amylase salivaire et contribuent, sur la surface dentaire, à l'agrégation microbienne. Certaines peuvent même se lier au lysozyme et à la peroxydase. Ces enzymes, réputées antimicrobiennes, favorisent aussi l'agrégation et l'adhérence bactériennes.

La glucosyltransférase extracellulaire bactérienne, active dans la pellicule, renforce aussi l'adhérence microbienne spécifique. (36)

Citons aussi d'autres éléments structuraux:
■ les protéines riches en proline acide,
■ la lactoferrine,
■ la fibronectine,
■ l'IgA à valeur de récepteurs spécifiques pour l'adhésion microbienne aux surfaces.

Ces récepteurs bactériens, les adhésines, sont électifs pour des sites, cryptitopes, démasqués par l'action d'enzymes lytiques sur ces protéines riches en proline acide.

Un système d'adhérence interbactérien fait intervenir:
■ les fimbriae, formant une couche bactérienne superficielle facultative riche en polysaccharides,
■ le glycocalyx, fixant le tétra-oxyde d'osmium en microscopie électronique.

Il en résulte que les propriétés et l'intégrité structurelle de la plaque dentaire sont subséquentes à la production de polymères extracellulaires par les bactéries liées au biofilm.

Cependant, 10 secondes suffisent aux bactéries pour coloniser des surfaces de la cavité buccale exposées:

Les bactéries orales présentant la capacité d'adhérer directement aux surfaces solides sans aucune pré-adsorption d'un film de conditionnement. (36)

### 1-1-1-3-3- Virulence et adhérence

Comme nous l'avons vu, l'organisation en biofilm accentue le caractère pathogène des bactéries.

Notons aussi que leur résistance contre les agents antimicrobiens- tant les fluorures d'amine ou stanneux que les antibiotiques- augmente un facteur de 100 à 1000, alors que l'efficacité antibiotique était testée, ces dernières décennies, sur des échantillons planctoniques.

Quoi qu'il en soit, ce biofilm buccal se développe à partir de bactéries de la salive qui contient 10⁸ à 10⁹ colonies CFU, seule phase planctonique hors mis la plaque «flottante » sous-gingivale.

80% des surfaces buccales colonisées (de 12 bactéries à plusieurs centaines par cellule eucaryote), concernent des tissus mous sans, pour autant, favoriser l'expression d'une pathogénicité.

Ces cellules et leurs bactéries sont ingérées après desquamation ou contribuent à développer plus rapidement les biofilms, à proximité de sites inflammatoires.

Des études récentes (4) ont suggéré que les différentes bactéries composant la flore buccale n'ont pas les mêmes cinétiques d'adhésion.

Pour qu'une bactérie puisse adhérer à une surface, il lui faut:
■ un site d'adhérence spécifique à cette surface compatible avec des moyens d'attache propres à cet organisme,
■ la réalisation de l'expression génétique de ces moyens d'attache,
■ la potentialité de fabriquer une matrice polysaccharidique.

L'analyse des mécanismes d'adhérence distingue *l'adhérence non spécifique* de *l'adhérence spécifique.*

La première, *réversible* sous l'effet de forces d'attraction hydrophobes, électrostatiques ou de mouvements browniens, entre bactérie et surface à coloniser, précède la seconde, *irréversible* dans des conditions physiologiques.

L'adhérence spécifique est assurée par la liaison entre récepteurs membranaires de la cellule-hôte et adhésines membranaires des bactéries. L'adsorption spécifique de certaines espèces à la pellicule acquise, constitue le processus initial de l'adhérence.

Il existe un autre système d'adhérence interbactérien qui fait intervenir les fimbriae formant une couche de surface riche en polysaccharides, le glycocalyx déjà mentionnés.

L'adhérence bactérienne présente une certaine spécificité vis à vis des protéines salivaires, facteurs de variabilité individuelle, d'où des différences de caractéristiques de colonisation. (36,110)

Il a été d'autre part rapporté des effets synergiques de T. denticola augmentant la virulence de P. gingivalis, dans des modèles animaux.

Ces deux souches provoquant une infection mixte déclenchent une réponse inflammatoire excessive. (8)

### 1.2 Bactériologie- Généralités

L'observation en microscopie à fond noir ou en contraste de phase ne manque pas de nous étonner en première intention, par le nombre, la grande diversité des organismes prélevés du biofilm sous-gingival et une cohabitation d'éléments si diversifiés dans un écosystème de fait extrêmement complexe. (33)

Sur les 700 espèces répertoriées dans la cavité buccale, 400 colonisent le biofilm sous gingival. (97)

Des sondes ADN ont été élaborées depuis, pour recenser des espèces jusqu'alors non encore cultivées. (78)

Les connaissances microbiologiques traditionnelles évolutives et la redéfinition de la plaque dentaire en biofilm microbien génétiquement régulé, ont considérablement contribué à modifier notre approche analytique des facteurs étiologiques locaux de la maladie parodontale (18).

Cette interférence génétique contribue à la "communication" inter espèces au cours de la formation du biofilm.

D'autres auteurs ont mis en évidence la capacité de certaines espèces à échanger des informations de cellule à cellule par transfert horizontal de gènes, favorisé par la proximité cellulaire avec, pour exemple notoire, l'acquisition de résistance à des antibiotiques, au sein d'une même espèce, ou d'une espèce à l'autre. (82).

Il est définitivement acquis aujourd'hui que les associations microbiennes du biofilm ne doivent rien au fait du hasard.

Certaines espèces investissent précocement l'espace parodontal et l'apprêtent pour d'autres espèces, comme des récepteurs à la colonisation.

De ce fait, nous observons des associations récurrentes. Ces interactions spécifiques répondent à des mécanismes mis en place grâce à des adhésines moléculaires et leurs récepteurs.Elles caractérisent certaines souches par leur relative électivité de pontage et d'autres par une plus vaste gamme de familles partenaires.

Au-delà de ces moyens de colonisation initiale (adhésion aux surfaces solides et inter espèces), des signaux intra et inter espèces, pourraient expliquer les séquences bactériennes successives qui conduisent à la formation du biofilm mature. (51)

L'occurrence des souches anaérobies dans l'établissement des parodontites a été mise en évidence depuis environ 30 ans en isolant *Tannerella forsythia* maintenant dénommée *Bacteroides forsythus*... (96)

Depuis, des synergies ont été décrites notamment avec *Fusobacterium nucleatum.*

A la fin des années 1970, le diagnostic étiologique des parodontites désigne *Aggregatibacter actinomycem comitens-* première bactérie du groupe des bactéries pathogènes parodontale- directement responsable de la parodontite agressive localisée. La régression de cette famille microbienne en favorise la guérison. (22)

### 1.2.1 Le milieu commensal

Une gencive cliniquement saine est très classiquement associée à des bactéries anaérobies facultatives et une faible proportion d'anaérobies strictes. Cette proportion se modifie de façon très variable si l'environnement (au sens extrêmement large) parodontal involue, jusqu'à favoriser l'apparition d'un état pathologique.

Au cours de la dernière décennie, les techniques de la biologie moléculaire - telles que séquençage génomique, phylogénie et identification des déterminants de virulence par la technique de l'ARNr 16S - ont largement été utilisées en parodontie. (97)

Ces techniques ont permis de mieux cerner la dimension microbiologique des maladies parodontales et d'établir l'étroite corrélation entre facteurs de virulence et expression de la maladie.

### 1.2.2 Les souches pathogènes

Il existe une corrélation directe entre la quantité de bactéries présentes et la sévérité de l'atteinte parodontale. De nombreuses études confirment que les patients atteints de parodontites agressives présentent un taux d'anticorps sériques ciblés sur les bactéries sous gingivales.

### 1-2-2-1- La répartition des familles pathogènes

La compréhension de la relation inter espèces bactériennes passe par l'approche de la biologie de la plaque dentaire sous gingivale et contribue à la planification des stratégies de son contrôle.

### 1-2-2-1-1- Les complexes microbiens sous gingivaux

Les familles microbiennes caractéristiques de l'écosystème sous gingival ont été regroupées en complexes décrits par Socransky (93').

Ces associations bactériennes, mises en évidence selon deux modalités d'étude - groupement et classement- répartissent en 5 groupes de 29 espèces, les 32 étudiées.

Les 3 dernières, sous forme libre, notamment *Aggregatibacter a.c sérotype b*., peuvent interagir avec les différents complexes énoncés.

### Le complexe rouge:

Porphyromonas gingivalis, Bacteroides forsythus, Treponema denticola,

### Le complexe orange:

Fusobacterium nucleatum ssp.(vincentii,polymorphum,periodonticum) Prevotella intermedia, Prevotella nigriscens, Peptostreptococcus micros, Eubacterium nodatum, Streptococcus constellatus, Campylobacter sp.(gracilis,showae,rectus)

### Le complexe jaune:

Streptococci sp. (mitis,oralis,sanguis: les plus fréquents et aussi streptomonas,gordoni,intermedius): S. sp.

### Le complexe vert:

Capnocytophaga sp. (gingivalis,sputigena,ochracea) Campylobacter concisus, Eikenella corrodens, Aggregatibacter actinomicetemcomitens sérotype a,

### Le complexe violet:

Actinomyces odontolyticus, Veillonella parvula, Actinomyces naeslundii serotype2 (Actinomyces viscosus), Selenomonas noxia, Aggregatibacter actinomycetemcomitens serotype b, ne sont pas regroupées avec les autres espèces, sans équivoque.

Le diagramme présenté par Socransky et collaborateurs est aussi un prétexte à d'autres études à venir, concernant les relations inter microbiennes sous gingivales.

### 1-2-2-1-2- Synergies

Certains auteurs (4) ont défini des *bactéries colonisatrices primaires*:

Elles contribuent à constituer le biofilm in vivo, dès ses premières heures de formation. Elles sont dépistées par technique d'hybridation ADN-ADN en damier. Ainsi, on identifie les germes des complexes violet et vert (mais aussi les Aggregatibacter ). Ils précèdent ceux du complexe jaune.

La faible proportion simultanée des bactéries des complexes orange et rouge évoque une cinétique d'adhésion variable selon les espèces.

Les bactéries du complexe rouge sont étroitement liées à celles du complexe orange (93').

Des interconnexions préférentielles existent aussi entre Capnocytophaga. sp. , la plupart des S. sp. , E. corrodens et Campylobacter concisus (soit entre les complexes jaune et vert).

Ces espèces sont fréquemment répertoriées simultanément et plus rarement en même temps que celles du complexe orange.

L'AAC serotype b n'a pas de corrélation avec C. ochracea.

En revanche, elle existe entre A.odontolyticus et V. parvula. Notons la synergie dissociée respectivement des complexes rouge et orange d'une part, et des complexes jaune et vert d'autre part.

Il apparaît clairement dans cette étude que les composants du complexe rouge sont rarement identifiés en l'absence de ceux du complexe orange.

La colonisation évolutive des bactéries du groupe orange s'accompagnerait d'un nombre grandissant de celles du groupe rouge.

La seule exception est l'intégration occasionnelle dans le complexe rouge de Eubacterium nodatum. P. gingivalis, T. denticola, B. forsythus sont fréquemment détectées simultanément dans les sites présentant une perte d'attache importante, dans les sites à poches profondes et gingivorragiques au sondage.

Leur proportion relative augmente sensiblement dans la plaque sous gingivale des patients atteints de parodontite.

Ce, plus particulièrement P. gingivalis et B. forsythus, non seulement dans des sites en phase active de destruction, mais aussi impliquées dans des parodontites débutantes, supposant un rôle dans l'initiation de la maladie. (8) B. forsythus, est unanimement reconnue "pathogène parodontale", désormais dépistée par technique immunologique.

Il semblerait selon certains auteurs (8) que B. denticola nécessite la présence de P.gingivalis pour s'établir.

Ces trois familles microbiennes associées entre elles ou à des bactéries commensales, ont capacité à constituer un biofilm.

Cette faculté d'adhérence hétérotypique permet le transport de P.gingivalis et B.forsythus par T.denticola, seule bactérie motile du complexe rouge, par un mécanisme de "piggyback".

Leur déplacement vers les zones plus profondes des poches parodontales favoriserait leur croissance surtout grâce à un environnement plus pauvre en oxygène. (8)

Nous avons signalé déjà l'influence de fusobacterium nucleatum sur la croissance de B.forsythus.

Ces souches, elles aussi, sont identifiées chez les patients présentant une parodontite agressive et/ou des poches profondes.

Quelques autres exemples d'interactions microbiennes sont illustrées sur le diagramme suivant:[d'après (4) et (97)].

### Types d'interaction:

**Adhérence : 1**
**Colonisation : 2**
**Nutrition : 3**
**Virulence : 4**

GFH:growth factor hémine: [**5**]
Adhésine (n heptasaccharides)[**6**]
Succinate: [**7**]
Acides organiques: [**8**]
Formate : [**9**]
Complexes : rouge [**R**], orange [**O**],jaune [**J**]
de Socransky

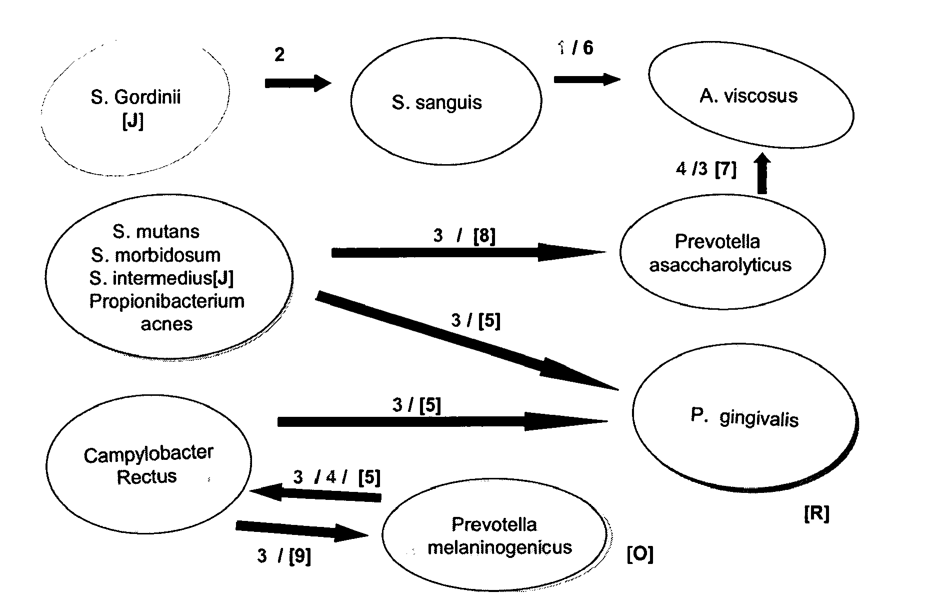

### 1.2.2. Les bactéries du complexe rouge se caractérisent aussi par des relations écologiques particulières, suggérées par des conditions de coexistence évoquées plus haut. (8)

### 1.2.2.1.3. Antagonismes

Pour exemples, les mutacines de S.mutans sont bactéricides pour S.viridans et A.viscosus (20).

Les sanguicines de S.sanguinis inhibent S.mutans et les mélanocines sont dirigées contre S.sanguis, mutans et aggregatibacter.

### 1-2-2-2- Conditions de prolifération microbienne

L'importance de certains composants salivaires a déjà été relevée depuis les cryptitopes de protéines fixées sur une surface qui révèlent autant de sites de fixation bactériens jusqu'alors masqués, à l'activité enzymatique (sialidases) qui démasque d'autres sites.

IgAs, mucines protéines riches en Pro (adhésions, Actinomyces), fibronectine, lysozyme et lactoferrine sont aussi impliqués.

Les agglutinines participent à l'adhérence de Actinomyces mais aussi de S.mutans. (4) Génétique et environnement interviennent dans l'adhérence et donc la stabilisation de souches au sein d'un écosystème qui prélude à leur prolifération.

Une infection virale herpétique associée à la maladie parodontale peut exposer la membrane basale par élimination des cellules endommagées et favoriser la fixation de bactéries sur ces nouveaux sites.

Autant de facteurs éminemment variables, selon les sujets et chez un même individu, soumis aux alea temporels et environnementaux.

La nutrition régule également la formation du biofilm.

Au même titre que sucrose, glucose, fructose favorisent la formation d'un biofilm par S. gordonii, la dégradation de l'urée (NH3, H2O et CO2) par Actinomyces lui apporte les nutriments utiles à sa prolifération.

Certaines interactions nutritionnelles entre bactéries du complexe rouge sont également favorisées par l'adhérence hétérotypique.

Un extrait cellulaire de B. forsythus stimule la croissance de P.gingivalis dans un milieu nutritionnel pauvre.

Certaines protéines sécrétées par P. gingivalis stimulent la croissance de T. denticola.

L'échange d'acides organiques définit une relation à bénéfice réciproque entre ces deux espèces.

Ces interactions d'adhérence et nutritionnelles contribuent à favoriser la formation d'un biofilm sous gingival pathogène chez les sujets présentant une maladie parodontale. (8)

D'autres enzymes (glycosyltransférases)-sous l'influence du pH, osmolarité, apport d'hydrates de carbone, disponibilité de récepteurs, potentiel redox- participent à la formation de glucanes, supports de l'adhésion. (4)

### 1.3 Conséquences des modes d'action microbiens et pathogénicité

Les bactéries anaérobies gram - du complexe rouge notamment, en exprimant de nombreux facteurs de virulence, colonisent l'espace sous-gingival, perturbent le système de défense de l'hôte, envahissent ou détruisent les tissus parodontaux, et/ou contribuent à promouvoir une réaction immunodestructrice du malade. (8)

Ce comportement manifeste un certain nombre de mécanismes pathogéniques qui s'expriment avec une acuité relativement variable au cours de la maladie parodontale.

Les bactéries du complexe rouge sont munies de nombreuses adhésines qui leur permettent d'adhérer sur différents types cellulaires, en plus de l'adhérence hétérotypique déjà décrite.

Citons l'adhérence aux cellules épithéliales, les fibroblastes, les érythrocytes, les leucocytes et de nombreuses protéines salivaires, du fluide créviculaire et de la matrice extracellulaire. (8)

### 1.3.1 Cellules épithéliales et matrice extracellulaire

P. gingivalis exprime au moins cinq haemagglutinines différentes (HagA, HagB, HagC, HagD, HagE) à sa surface cellulaire, impliquées dans sa liaison aux cellules de l'hôte.

T.denticola est munie de protéines LrrA (leucine-rich repeat protein) et Msp (major sheath (or surface) protein), grâce auxquelles il se lie aux cellules de l'hôte et aux protéines de la matrice extracellulaire.

B.forsythus a une couche S contribuant à l'adhérence de la bactérie aux surfaces cellulaires.

Rappelons que les protéases de P. gingivalis et T. denticola démasquent les sites cryptitopes à la surface des cellules eucaryotes, créant autant de récepteurs à l'adhésion des parodontopathogènes.

L'adhérence de P. gingivalis aux cellules épithéliales provoque la formation d'une invagination de la membrane plasmique qui entoure la bactérie, déclenchant son internalisation.

P. gingivalis a la capacité de se multiplier à l'intérieur des cellules épithéliales.

D'autres auteurs (8) ont démontré que cette bactérie présente aussi la faculté de se propager d'une cellule épithéliale à une autre, sans passer par l'espace extracellulaire.

Sa dissémination dans les tissus parodontaux est alors assurée, protégée de la réponse de l'hôte.

Des études in vitro, (8) confirmées par des observations in vivo, suggèrent que P. gingivalis et T. denticola ont capacité à franchir la membrane basale et pénétrer profondément dans le chorion parodontal.

Même chez des sujets sains, ces souches du complexe rouge ont été observées à l'intérieur des cellules épithéliales buccales.

B.forsythus semble être la bactérie la plus commune à la surface et à l'intérieur des cellules épithéliales issues de poches parodontales.

### 1.3.2 Invasion bactérienne et altération du métabolisme cellulaire de l'hôte

In vitro, la pénétration de P. gingivalis et B. forsythus dans le cytoplasme des cellules épithéliales est à l'origine d'un "dialogue moléculaire" entre bactéries et cellules eucaryotes.

L'adhérence provoque d'une part, l'activation de différentes voies de signalisation cellulaire chez la cellule hôte (activation des MAP kinases, phosphorylation des protéines, augmentation du flux calcique, réorganisation du cytosquelette) et, d'autre part, modifie l'expression de la traduction génique chez la bactérie. (8)

P. gingivalis et T. denticola produisent des vésicules de petite taille (50nm), à partir de leur membrane externe, renfermant leurs principaux facteurs de virulence connus. Ces microstructures assurent une diffusion de ces principes de virulence dans des sites inaccessibles à des cellules entières au détriment des tissus parodontaux.

L'invasion tissulaire et l'internalisation des bactéries du complexe rouge sont largement impliquées dans leur pouvoir pathogénique à effet délétère et altèrent l'homéostasie des tissus.

Certains pathogènes comme Fusobacteria, Peptostreptococcus micros et T. denticola, métabolisent GSH (glutathion réduite, voir stress oxydatif) en sulfite d'hydrogène toxique. (11)

### 1.3.3 La barrière endothéliale

L'épithélium de jonction a été comparé à l'épithélium des cryptes intestinales et il peut se comporter comme un épithélium réticulé, définissant un environnement propice aux processus locaux de reconnaissances immunitaires. Le nombre de desmosomes et de jonctions serrées de connexion de l'épithélium de jonction sont petits et les espaces intercellulaires restent larges. Ces caractères favorisent l'infiltration leucocytaire et la diffusion antigénique depuis l'espace sulculaire au travers de la lamina propria gingivale, richement vascularisée.

30.000 neutrophiles migrent en moyenne par minute à travers un épithélium de jonction d'une gencive saine.

La migration à travers une barrière, épidermique ou autre, est régulée par l'expression d'une molécule d'adhésion intercellulaire-1 (intercellular adhesion molecule-1 : ICAM-1).

Cette molécule semble être un moyen de guidage important des neutrophiles vers le milieu sulculaire.

P. gingivalis empêcherait la migration des polymorphonucléaires neutrophiles à travers l'endothélium en inhibant la production de IL8 et ICAM1 (intracellular adhesion molecule). (8)

### 1.3.4 Activité enzymatique des bactéries

Les souches du complexe rouge jouent de multiples rôles dans la pathogénèse des parodontites, en particulier, sous la forme d'un fort pouvoir protéolytique.

Les protéases produites par ces bactéries interviennent au niveau de l'adhérence, la nutrition, la dissémination des bactéries et contribuent à la perturbation des défenses de l'hôte et la destruction des tissus parodontaux. (8)

P. gingivalis est, parmi les bactéries pathogènes, la plus virulente par l'activité des gingipaïnes, trois cystéines protéases, en rapport direct avec les pertes d'attache observées chez les patients à parodontites chroniques et donc les destructions tissulaires.

Ces gingipaïnes augmentent la perméabilité vasculaire en activant le système kallicréïne- kinine et perturbent la coagulation sanguine en dégradant le fibrinogène, la fibrine et le facteur X.

Ces protéases, en outre, dégradent les immunoglobulines (IgA, IgG, IgM).

De ce fait, les bactéries échappent à l'opsonisation par les cellules phagocytaires et à l'action du système du complément.

Elles atteignent aussi certains récepteurs membranaires, les protéines du complément, notamment en clivant la protéine C3 et en inactivant le récepteur de la protéine du complément C5a, présent à la surface des neutrophiles. La destruction de ce dernier récepteur joue un rôle majeur dans le chimiotactisme des neutrophiles au site de l'infection, provoquant une perturbation des fonctions phagocytaires.

Enfin, elles provoquent la dégradation des cytokines pro-inflammatoires, incluant l'IL-1β, le TNF-α et l'IL-6 et les chimiokines, notamment IL-8 et le RANTES. (8, 70)

Elles contribuent ainsi à contourner les défenses immunitaires de l'hôte.

La dégradation des cytokines par P. gingivalis peut être massive, dépend à la fois de la concentration microbienne et de la souche utilisée.

Cette dégradation conduit à une forte perturbation des réactions inflammatoires locales et une rupture du gradient de chimiokines à proximité de la plaque sous-gingivale ayant pour effet d'inhiber l'afflux des leucocytes au site de l'infection.

Les gingipaïnes de P. gingivalis et la dentilisine de T. denticola dégradent de nombreux composants de la membrane basale et de la matrice extracellulaire, tels que le collagène de type IV, la laminine et la fibronectine.

Les gingipaïnes peuvent aussi cliver le syndecane-1 de la surface des cellules épithéliales gingivales, un corécepteur de divers facteurs de croissance et de molécules de la matrice extracellulaire, impliqué notamment dans l'adhésion des cellules à la matrice.

Les protéinases de ces deux mêmes familles bactériennes peuvent aussi dégrader les jonctions intercellulaires des cellules épithéliales, dégradant la perméabilité de la barrière épithéliale et l'intégrité des tissus parodontaux.

Ces bactéries dégradent de nombreux inhibiteurs de protéases de l'hôte (α1-antitrypsine, antichymotrypsine, α2-macroglobuline, antithrombinelll, antiplasmine et cystatine C), impliqués dans la régulation de l'activité de nombreuses enzymes protéolytiques de la réponse inflammatoire.

D'autres récepteurs cellulaires, incluant le CD14 (récepteur des LPS) et le récepteur de IL-6 (IL-8R) sont clivés sous l'action des gingipaïnes, perturbant les réactions inflammatoires locales induites par le LPS et l'IL-6.

L'outillage enzymatique des souches asaccharolytiques de ce complexe, leur permet de répondre à leur besoin nutritif à partir des protéines tissulaires et plasmatiques. Ce moyen de nutrition leur assure les moyens de prolifération dans la poche parodontale.

Ces bactéries, par le biais des haemagglutinines et leur activité hémolytique, se lient aux érythrocytes et les détruisent.

La libération d'hémoglobine constitue une source de fer indispensable à la croissance des parodontopathogènes.

P. gingivalis est doté, de surcroît, de collagénases dégradant le collagène de type I, participant aussi, à ce titre, activement à la destruction tissulaire.

Rappelons aussi, pour exemple, le rôle de protéoglycanes comme l'héparan sulfate des cellules endothéliales.

Ces molécules contribuent à inhiber la coagulation intravasculaire: le composant glycosaminoglycane de cette protéoglycane accélère l'inactivation de la thrombine par inhibition de la protéase. (F14) Les bactéries munies de telles enzymes pourraient jouer un rôle similaire.

Une étude (F15) a mis en évidence le comportement différentiel des bactéries vis-à-vis de leur action enzymatique de dégradation du milieu extracellulaire.

Il s'avère que des germes gram+ (B. mutans et B. matruchotii) ne présentent aucune activité enzymatique vis-à-vis de la fibronectine.

Il en va de même pour Haemophilus actinomycetemcomitans, gram-. En revanche, des germes gram- (B. intermedius, B. gingivalis) manifestent une capacité à dégrader ce substrat, particulièrement B. gingivalis pour laquelle cette activité est due, entre autre, à une cystéine protéinase.

Les enzymes, identifiées par test d'inhibition exprimée par B. intermedius, sont des protéinases à sérine et à cystéine.

Rappelons-nous des propriétés singulières de la fibronectine, notamment sa participation à la cohésion et structuration spatiale des tissus conjonctifs. Cette activité microbienne laisse alors présumer des conséquences fonctionnellles des parodontopathies par les impacts notamment destructeurs des tissus de soutien.

### 1.3.5 Parmi les cellules, fibres, et molécules interstitielles, la fibronectine

La fibronectine est une des glycoprotéines de structure, composant la matrice extracellulaire dont la fonction phylogénétique n'est plus à décrire, quant à sa contribution à la régulation des comportements entre les cellules regroupées en organes et appareils (développement, migration, prolifération, formes et fonctions métaboliques).

Cette classe de macromolécules dont la complexité et la diversité sont mieux connues aujourd'hui, intervient, tant dans la structure matricielle, qu'au niveau des interactions cellules- matrice.

Elles sont considérées comme des protéines d'ancrage des cellules au sein des tissus conjonctifs et contribuent à la stabilité tridimensionnelle de la structure.

La fibronectine existe in vivo sous deux formes:
Une, soluble, présente dans le plasma et d'autres liquides biologiques (FNp),
Une, insoluble, (FNc) distribuée dans les tissus conjonctifs lâches et diverses membranes basales.

Elle est synthétisée par de nombreux types cellulaires.

De haut poids moléculaire (450000), les fibronectines sont formées de deux sous-unités similaires, pas nécessairement identiques.

Par séquençage d'ADNc, FNp et FNc proviennent d'un seul gène par épissage alternatif du premier transcrit. L'omission d'un exon ou la subdivision d'un autre exon peut être à l'origine des différences entre FNp et FNc et entre les sous-unités de FNp.

Malgré des différences de nature biophysiques et biochimiques, ces deux formes partagent des caractéristiques communes, notamment un caractère immunologique commun vis-à-vis d'anticorps polyclonaux, des interactions avec les collagènes, la fibrine, des protéoglycannes, des éléments du cytosquelette, des bactéries et les surfaces cellulaires. Les domaines médiant ces interactions ont été localisés.

Un domaine circonscrit contient un site récepteur pour une transglutaminase qui permet un pontage covalent avec le collagène, la fibrine et des bactéries. L'affinité pour la fibrine et pour l'héparine est distribuée sur deux sites. Un domaine adjacent au domaine N-terminal médie une interaction non covalente avec le collagène.

Dans la zone centrale de la molécule, se trouve le site de liaison avec les surfaces cellulaires.

Ce site par exemple reconnaît un petit protéoglycan présent sur le fibroblaste, la décorine (ou PG40 ou PGII).

Le rôle de la fibronectine dans l'adhésion cellulaire a été clairement établi.

La fibronectine en solution existe sous une forme monomérique et fibrillaire. Chaque sous-unité de la molécule contient des sites de liaison complémentaires qui s'attirent les uns les autres.

Dans la fibronectine soluble, ces sites sont saturés par des interactions intramoléculaires et, dans la forme fibreuse, par des liaisons intermoléculaires.

Des sites d'affinités impliqués dans l'auto-association de la fibronectine ont été identifiés dans les régions N- et C- terminales des sous-unités.

L'analyse des interactions et de la structure de la fibronectine laisse présumer l'action de cette molécule comme ligand entre les macromolécules de la matrice d'une part, entre ces macromolécules et les surfaces cellulaires, d'autre part.

Certaines fonctions biologiques ont été mises en évidence in vivo et in vitro, notamment un rôle dans l'adhésion des cellules à leur substrat, un rôle dans la morphogénèse des tissus conjonctifs et dans la réparation tissulaire.

In vitro, une altération de la répartition quantitative de fibronectine dans des fibroblastes transformés par des virus oncogènes a modifié la forme devenue plus arrondie de ces cellules et leur comportement social en perdant l'inhibition de contact.

Réinjecter FNc dans de telles cultures a restauré une morphologie normale cellulaire, les propriétés d'adhésion et leur cytosquelette.

In vivo, dans des carcinomes infiltrants de glandes mammaires, au niveau des membranes basales périvasculaires et périglandulaires, dans le tissu conjonctif lâche et à la surface des cellules épithéliales, on observe une disparition précoce de la fibronectine des membranes cellulaires, sa fragmentation puis sa disparition des membranes basales périglandulaires.

Le stroma périglandulaire présente une immunofluorescence très intense pour la fibronectine. De nombreuses hypothèses plausibles ont été évoquées pour expliquer ce phénomène. Quoi qu'il en soit, on observe une profonde perturbation des relations cellules-matrice dans les tumeurs humaines solides.

Un domaine de la macromolécule a été mis en évidence par digestion protéolytique comparée de la fibronectine, détectable dans les tissus foetaux et tumoraux et pas dans la fibronectine de l'adulte sain: le domaine oncofoetal.

Ces exemples de dérèglement mettent l'accent sur le besoin de préserver le fragile équilibre de l'écosystème biologique dont la moindre perturbation peut avoir des conséquences aux effets incalculables.

D'autres illustrations de modifications des interactions cellules-matrices s'appuient sur le phénomène de vieillissement et des pathologies comme le diabète.

Le génome cellulaire détermine le programme de biosynthèse, âge-dépendant.

Il en résulte un changement continuel de composition de la matrice extracellulaire de tous les tissus et organes.

Les interactions cellules-matrice sont interdépendantes et vraisemblablement sous l'influence étroite des GPS.

La fibronectine sécrétée par des cellules, vieillies par repiquages successifs en culture, présente un même défaut qui provoque l'altération de la capacité d'adhérence cellulaire.

Ces formes structurales et fonctionnelles différentes de la fibronectine de cellules âgées manifestent des interactions modifiées notamment avec les collagènes natifs de types I et III.

L'étude de cette molécule par l'activité protéolytique, a démontré l'existence de sites hautement spécifiques, cibles des enzymes. Les protocoles mis en place ont préservé l'activité biologique de chaque portion isolée. Il en résulte une haute définition des domaines structuraux de la fibronectine qui interagissent avec le collagène, les surfaces cellulaires, l'héparine, la fibrine.

Cette analyse confirme l'utilité majeure de cerner au mieux l'activité enzymatique des bactéries pathogènes impliquées dans les maladies parodontales. Les risques de dénaturation de telles molécules mettrait alors en cause l'intégrité tissulaire et favoriserait l'accélération du processus agressif et destructeur de la maladie.

Certaines études ont montré que la fibronectine fixée sur les cellules épithéliales constitue une barrière protectrice contre l'adhérence de certaines bactéries gram-.

L'activité de liaison à la fibronectine est considérée comme un des mécanismes de colonisation microbienne.

L'apparition de fimbriae sur P.gingivalis serait liée à des modifications de milieu, tels que la présence d'anticorps, d'hémine antibactérienne ou d'antibiotiques, rejoignant l'idée de quorum sensing évoquée plus loin.

### 2-LA VOIE 2

### 2.1- La salive et ses composants

Certains composants de la salive lui confèrent un rôle antibactérien:
- Le lysozyme, enzyme bactériolytique ciblant la paroi microbienne, contribue à la défense non spécifique immunitaire.
- La lactoperoxydase, favorise la transformation de l'ion thiocyanate SCN, sous l'effet de peroxyde d'hydrogène, en ion thiocyaneux très bactériotoxique.
- La kallicréine hydrolyse le kininogène en kinine, médiateur de l'inflammation. Elle active cyclooxydase et phospholipase A2, qui jouent un rôle dans la synthèse des prostaglandines PGE2.

### 2-1-1- Récepteurs et adhésines

2.1. Les récepteurs, génétiquement déterminés, dépendent essentiellement de l'hôte. Leur expression, synthèse d'adhésines dépendant de facteurs périphériques, permet la colonisation des surfaces de l'hôte, dans la stricte mesure où les récepteurs aux adhésines sont démasqués, au besoin par un outillage enzymatique émanant de la bactérie. (4)

### 2.1.2. Biofilm et inflammation

Le biofilm, indirectement par sa composition bactérienne, agit sur le processus inflammatoire et constitue un élément à prendre en considération dans l'évaluation de l'intensité de la réaction inflammatoire.

Les caractéristiques biophysiques et biologiques du biofilm, largement évoqués précédemment, contribuent aussi à la hauteur de la réaction de ce processus.

### 2.1.3. Quorum sensing

### Définition:

Capacité des bactéries, à répondre ensemble à des modifications environnementales, par régulation de expression génétique, selon la densité de population microbienne, tant gram + que gram-. (4)

Des molécules codées génétiquement, les auto-inducteurs, sont sécrétées par les bactéries dans le milieu extracellulaire.Ce partage d'informations auto-induites, permet à la population bactérienne une coordination d'activité, selon sa concentration.

Des différences comportementales existent, pour une même bactérie, selon qu'elle est en phase planctonique ou en biofilm. La suppression du gène codant ces inducteurs semble perturber le gène codant les enzymes nécessaires à la formation de composants du biofilm.

Par exemple, l'inhibition du gène luxS codant l'auto-inducteur AI-2 (régulant les gènes codant pour la glucosyltransférase) provoquerait pour S.mutans une incapacité relative à former le biofilm, probablement par augmentation de l'auto-agrégation de la bactérie.

Les interactions génétiques peuvent concerner des espèces voisines. L'inactivation de luxs n'empêcherait pas l'expression de gènes codant pour des adhésines par S.gordonii. Cette bactérie hyporégule, par ailleurs, l'expression de plusieurs gènes impliqués dans le métabolisme des hydrates de carbone composant le biofilm.

Il semblerait suffisant, selon d'autres résultats, que AI-2 soit présent dans le milieu, quelle qu'en soit la source, pour rétablir la capacité à former un biofilm.

Un autre auto-inducteur, seulement pour les gram +, le CSP (competent stimulating peptide), favoriserait également la croissance d'un biofilm de S. intermedius.

La stabilité de l'adhérence- via la codification génétique de la matrice du biofilm- nécessite une communication par messages transmis entre les membres de la communauté microbienne.

Cette stabilisation acquise favorise aussi l'apparition de sites électifs pour des souches colonisatrices secondaires, incapables de se fixer au premier abord, transformant la flore commensale en pathogène opportuniste et engageant l'expression de la maladie.

### 2-2- Les réponses immunitaires

Rappelons, pour mémoire, les données fondamentales:

L'organisme s'est doté d'un système qui lui permet de conserver son individualité et son intégrité:
c'est le système immunitaire, qui distingue le soi du non-soi, élimine ou neutralise les substances étrangères qui peuvent s'y introduire, en particulier les agents infectieux.

Conserver son intégrité, c'est aussi empêcher que des lésions importantes n'entraînent la fuite du soi dans le milieu extérieur:
le système de la coagulation, comme le système immunitaire, participe ainsi au maintien de l'individualité, ce qui explique les relations qui se sont établies entre les deux systèmes.

L'organisme tolère cependant la présence de bactéries à la surface de sa peau et de ses muqueuses, en établissant des relations de commensalisme, voire de symbiose.

Cependant, ces bactéries peuvent profiter de l'opportunité que leur offre une défaillance du système immunitaire pour provoquer une infection grave appelée *infection opportuniste*.

Le système immunitaire assume donc vis-à-vis des bactéries une double fonction:
■ Contenir les bactéries commensales qui peuvent devenir, à l'occasion, des bactéries pathogènes opportunistes.
■ S'opposer à la pénétration de bactéries virulentes, qui sont des bactéries pathogènes spécifiques ou avérées.

### 2-2-1- Non spécifique

L'immunité non spécifique est polyvalente. Elle existe avant tout contact et manifestation de l'agent infectieux. Sa mise en oeuvre est donc immédiate.

Quel que soit l'agent infectieux (virus, champignons, bactérie, parasite), le mode d'action est le même: phagocytose initiée et entretenue par la réaction inflammatoire.

### 2-2-2- Spécifiques

L'immunité spécifique est, par définition, daptée à chaque agent infectieux.

Elle nécessite une reconnaissance préalable de l'agresseur: sa première mise en oeuvre est donc retardée (phase de latence de la réaction *primaire*). Ses modalités sont variées et font appel à des médiateurs cellulaires, les lymphocytes T et B.

Contrairement à l'immunité non spécifique, elle présente la faculté de garder en mémoire le souvenir de la première agression.

Une agression ultérieure, par le même agent infectieux, provoque une réponse immunitaire plus rapide, plus adaptée et plus intense (réaction *secondaire*).

Immunités non spécifique et spécifique sont intimement liées.

La première est indispensable à l'activation de l'immunité spécifique en lui présentant les antigènes.

En retour, les produits de l'immunité spécifique cellulaire et humorale améliorent les performances de l'immunité non spécifique.

La susceptibilité à la parodontopathie et la sévérité de la réaction inflammatoire,sont une conséquence du niveau de la réponse immunitaire du malade aux bactéries pathogènes. L'interaction entre les produits bactériens et les tissus parodontaux se manifeste par l'expression de molécules d'adhésion et la production de cytokines pro-inflammatoires. Les neutrophiles sont amenés à franchir la barrière endothéliale vers les tissus affectés et dans le sulcus en franchissant l'épithélium de jonction. En présence constante de bactéries dans le sillon gingival, lymphocytes et macrophages deviennent les cellules prédominantes dans l'infiltrat inflammatoire. Chez les sujets prédisposés à la maladie parodontale, cela se traduit par une perte d'attache et d'ancrage osseux.

Les LPS bactériens et les cytokines comme II-1 , TNF-α, PGE-2 inhibent les gènes de production de collagène et activent les gènes des métalloprotéinases des cellules épithéliales, des cellules endothéliales et des fibroblastes, ce qui contribue à la désorganisation de la matrice extra- cellulaire.

La progression de la maladie est la conséquence de la rupture de l'homéostasie provoquée par la présence continuelle des bactéries pathogènes, les niveaux croissants de cytokines pro-inflammatoires, des métalloprotéinases de la matrice et PGE₂ et les niveaux décroissants de II-10, TGF-β (Tranforming growth factor β, et des inhibiteurs tissulaires de métalloprotéinases.

L'équilibre entre ces différents médiateurs conditionne les caractéristiques de la réaction inflammatoire. (41)

Le rôle fondamental des cellules T a été suggéré depuis déjà 30 ans, dont le fonctionnement est régulé de façon prédominante par les cellules B. Ces cellules T semblent jouer un rôle central dans le contrôle de l'auto-immunité. (25)

Il semblerait que P. gingivalis dérégule environ un millier de gène des cellules T, et de ce fait, constitue la pièce centrale d'un mécanisme qui invalide un équilibre ou une inertie entre la flore du biofilm et l'hôte:
le rôle des cellules T serait plus homéostatique que défensif ou destructeur. (25)

### 2-3- Les processus inflammatoires

Les réponses immunitaires et inflammatoires à la flore et aux virus colonisant le parodonte et tissus associés, mettent en oeuvre les systèmes de défense de la circulation systémique et périphérique.

Cela engendre des interactions complexes bidirectionnelles hôte-microflore, faisant intervenir les facteurs cellulaires et humoraux et le système des cytokines, interleukines et facteurs de croissance.

Alors que l'agent primaire est quasi-spécifique, à prédominance d'anaérobies gram- ou anaérobies facultatives, du biofilm sous-gingival, l'essentiel de la destruction des tissus parodontaux est provoqué par une réponse de l'hôte inadaptée à ces micro-organismes et leurs produits. (11)

### 2-3-1- Marqueurs CRP " C Reactiv Protein"

La CRP est une protéine synthétisée par le foie et doit son nom au fait de pouvoir précipiter avec le polysaccharide C du pneumocoque.

Son taux sérique, à l'état de trace (< 12mg/l), s'élève rapidement dès le début de la réaction inflammatoire, sous l'influence notamment de l'Il 1, sécrétée par le macrophage activé.

Ce taux se normalise quand la chaîne réactive inflammatoire est contrôlée.

La CRP se fixe à de nombreuses bactéries et augmente la phagocytose. Elle active la voie classique du complément, favorise la phagocytose en générant le facteur C3b.

L'augmentation de ce marqueur de l'inflammation a été associée à un haut risque de maladies cardiaques coronaires (CHD).

Même si les motifs de cet accroissement ne sont pas totalement élucidés, il semblerait que le stress oxydatif, que nous évoquerons plus loin, soit un élément à prendre en considération dans le taux de CRP.

Il contribuerait ainsi à favoriser le processus inflammatoire pro-athérrosclérotique, accompagnant l'installation des CHD. (1)

### 2-3-2- Cytokines

Les cytokines sont des macromolécules dont la fonction est de neutraliser l'infection, par des actions multifocales.

Pyrogène-endogène, elles agissent sur le centre de régulation thermique de l'hypothalamus, en provoquant une hyperthermie, qui a pour effets:
- d'inhiber la croissance bactérienne et autres agents infectieux par blocage des systèmes enzymatiques,
- d'augmenter la mobilité des granulocytes et leur capacité bactéricide,
- d'accroître la production d'interféron, qui, une fois produit par les cellules contaminées, diffuse et se fixe à des récepteurs spécifiques des cellules non infectées.
- Alors, il dé-réprime la synthèse de plusieurs systèmes enzymatiques, qui s'activent si l'agent pénètre dans la cellule et empêchent son information génétique de s'exprimer.

### 2-3-2-1- Interleukines

Les interleukines IL-1α, IL-1β, et IL-6 sont, au même titre que le TNF-α, mentionnées de façon récurrente dans les maladies inflammatoires.Le polymorphisme, au niveau des formes allèliques des gènes codant pour ces cytokines pro-inflammatoires, peut déclencher une amplification génique qui peut prédisposer le patient à l'expression de la maladie parodontale.L'incidence de ce polymorphisme a été abordé dans de nombreuses études (12)

### 2-3-2-2- TNF-α (Tumor Necrosis Factor α) ou cachectine

(7) Historiquement, la régression spontanée de tumeurs, a été mise en évidence en 1868 par le physicien allemand P.BRUNES, chez des patients présentant une infection aigue, la tuberculose.

En 1944, un des inducteurs principaux du TNF, l'endotoxine, un polysaccharide bactérien, est isolé des bactéries gram- par Murray SHEAR. Ce polysaccharide induit la nécrose hémorragique des tumeurs.

En 1962, O'MALLEY rapporte la production d'un facteur sérique chez les souris normales stimulées par l'endotoxine, le sérum de ces mêmes animaux, pouvant induire la nécrose, lorsqu'il est administré à des porteurs de tumeurs.

Il a été nommé tumor necrosis serum(TNS), puis renommé en 1975 Tumor Necrosis Factor (TNF).

Son identité moléculaire n'a été révélée que 10 ans plus tard, grâce au clonage et séquençage de son ADNc.

Un facteur cytotoxique a été alors isolé d'une lignée de cellules B lymphoblastoïdes et nommé lymphotoxine.

Il a été renommé plus tard TNF-β. L'analyse de leur séquence peptidique et de leur ADNc a révélé que le TNF-α et le TNF-β ont 50% d'homologie en acides aminés.

Aujourd'hui, le groupe des TNF s'associe à des facteurs membranaires ou solubles et régulent les systèmes de défense immunitaire, la survie cellulaire et l'organogenèse.

Il recense plus de 40 systèmes ligands-récepteurs, formant une famille homologue à celle des récepteurs du TNF. Les ligands, soit se combinent à la membrane, à un ou plusieurs récepteurs spécifiques à la surface des cellules, soit sont sécrétés.

Cette famille est définie par des homologies de séquences et de structures dans les ligands et récepteurs, par rapport au système TNF-TNFR. Les ligands, tout comme le TNF, sont des protéines transmembranaires de type II, avec un domaine homologue au TNF (THD) en C-terminal. Ces THD se replient en «sandwich antiparallèles», qui forment un trimère, chaque ligand ayant ainsi trois sites de liaison.

Le ligand étant multivalent, les récepteurs se regroupent, afin de permettre la liaison du ligand et la transduction du signal.

De nombreuses cellules produisent le TNF-α. Les monocytes et les macrophages sont les principales sources de TNF-α. Mais aussi les lymphocytes T et B, les cellules natural killer (NK), les polynucléaires (surtout les neutrophiles), les mastocytes, les fibrocytes, les chondrocytes, les astrocytes, les cellules microgliales, les cellules endothéliales activées, les kératinocytes et les cellules épithéliales digestives.

Un des aspects de la signalisation intracellulaire de la production de TNF-α est le polymorphisme du promoteur du TNF-α.

Le promoteur du TNF-α possède des sites de fixation pour différents facteurs de transcription, principalement NF-κβ et AP-1, AP-2, NF-AT (nuclear factor of activated T cell), CRE (cyclic adenosine phosphate response element),... . (7)

Ces différents facteurs de transcription peuvent varier et interagir de façon différente, selon le type de stimulus et le type de cellule stimulée. Le polymorphisme du promoteur du TNF-α influence les capacités individuelles de synthèse du TNF et donc potentiellement la susceptibilité aux maladies inflammatoires (production élevée de TNF) et aux risques d'infection (faible production de TNF).

### Mentionnons aussi la voie du nuclear factor-κβ (NF-κβ):

La transduction du signal fait suite à la fixation du stimulus physique (UV, RX, chaleur), chimique, immunologique, infectieux) sur son récepteur membranaire. Cette activation agit sur NF-κβ qui, à l'état basal, est séquestré dans le cytoplasme des cellules non activées par les molécules inhibitrices de la famille Iκβ.

L'activation cellulaire provoque une phosphorylation de l'inhibiteur par un complexe Iκκ.

Iκβ ainsi phosphorylé libère NF-κβ, puis est dégradé dans le protéasome. NF-κβ libre transloque alors vers le noyau où il exerce son rôle d'activateur de la transcription. (7)

Enfin, la voie des "mitogen activated protein kinase (MAPK) où la liaison du stimulus à son récepteur induit le recrutement de protéines intracellulaires (TRAF, TRADD et RIP), qui déclenchent une cascade de phosphorylations, via JNK et p38.

Les MAPK favorisent l'activation du facteur de transcription AP-1.

### 2-4- Génétique et cascades réactionnelles. TNF α et interleukines

La variation génétique repose sur des différences entre individus, dans la séquence nucléotidique de leur génome.

Même si les changements observés et exprimés dans l'ADN ne sont pas forcément délétères, leur manifestation la plus évidente et souvent la plus extrême, en est la maladie génétique, observée sur un fond normal de variabilité génétique. (101)

Le bon déroulement de l'ensemble des processus métaboliques et du développement de tout organisme est assujetti au fonctionnement de nombreuses protéines (enzymes ou protéines de structure), synthétisées dans les cellules. La relation princeps, entre les gènes et les protéines, est définie par la séquence codante des bases au niveau d'un gène donné.

Un changement dans une séquence de bases (mutation), peut conduire à la formation d'une protéine variante, qui peut avoir des propriétés altérées, en raison des changements au niveau de sa structure.

Cependant, d'autres changements au niveau de l'ADN peuvent n'avoir aucun effet phénotypique.

Soit parce que le changement n'altère pas la séquence primaire des acides aminés d'un polypeptide,
soit parce que le changement de la séquence des acides aminés survient dans une région non critique du polypeptide.

Toutes les protéines variantes n'entrainent donc pas nécessairement des conséquences cliniques.

Au contraire, un grand nombre de protéines fonctionnelles existent sous deux ou plusieurs formes relativement fréquentes, distinctes génétiquement. Une telle situation est appelée polymorphisme.

Un grand nombre de loci possèdent un certain nombre d'allèles, relativement fréquents, qui permettent de classer les membres d'une population, dans des catégories phénotypiques distinctes.

Le polymorphisme génétique est défini comme la présence d'allèles multiples à un même locus, lorsqu'au moins deux allèles présents ont une fréquence supérieure à 1%.

Donc des loci polymorphes sont des loci pour lesquels au moins 2% de la population est hétérozygote.

Cependant, puisqu'un grand nombre de loci polymorphes sont caractérisés par un grand nombre d'allèles différents, la proportion des hétérozygotes à certains loci est beaucoup plus grande.

Par convention, les allèles avec une fréquence inférieure à 1% sont appelés des *variants rares.*

Ces dernières années, de tous les loci testés vis-à-vis de ce caractère variant, pour nombre de protéines ou autres enzymes humains, un tiers environ a montré un polymorphisme, détectable dans au moins un groupe ethnique ou un groupe racial donné et, souvent, dans tous les grands groupes de population(101).

La présence très répandue de tels variants suggère que chaque individu est vraisemblablement hétérozygote pour un grand nombre de loci différents.

Quelle est la proportion de loci, chez un individu donné, pour lesquels il existe deux allèles différents, chacun spécifiant un produit génique de structure distincte?

Il a été estimé, à partir d'études d'un grand nombre d'enzymes, que chaque individu est vraisemblablement hétérozygote, pour environ 6% de l'ensemble des loci codant pour des enzymes.

En tenant compte des mutations qui ne peuvent être dépistées par électrophorèse, on peut estimer que chaque individu est vraisemblablement hétérozygote à environ 12 à 18 % de tous les loci codant, pour les polypeptides.

Ce chiffre est parfois appelé hétérozygotie moyenne et souligne le degré important d'individualité biochimique qui existe à l'intérieur de l'espèce humaine. Les polymorphismes sont utiles, en tant que "marqueurs" génétiques, permettant de distinguer au cours des études familiales des formes différentes d'un gène.

Ces marqueurs génétiques présentent une aide précieuse:
■ Localisation des gènes sur les chromosomes par l'analyse de liaison.
■ Diagnostic pré-symptomatique et prénatal des maladies génétiques.
■ Détection des hétérozygotes porteurs de maladies génétiques.
■ Evaluation des individus ayant des risques, élevés ou faibles, de prédisposition aux maladies communes de l'adulte, telles que la maladie cardiaque coronarienne, le cancer et le diabète.
■ Test de paternité et de médecine légale.
■ Etude des donneurs et des receveurs pour transplantation de tissu et d'organe. (101)

Bien qu'initialement découvert au niveau des groupes sanguins, le polymorphisme est très largement répandu et constitue un phénomène universel, qui a été documenté pour un très grand nombre de loci codant, pour un grand nombre de produits protéiques différents.

Les allèles mutés, responsables de maladies génétiques sévères, ne forment souvent que la forme la plus évidente de diversité génétique. Il apparaît qu'un grand nombre de protéines sont observées dans différentes populations, sous des formes relativement fréquentes et clairement distinctes.

Certains de ces allèles polymorphes ont une importance clinique, soit individuellement, soit en combinaison avec un allèle rare et délétère.

Pour illustration, nous pouvons évoquer la variation génétique au locus de la galactosémie, ou la variation génétique au locus de l'alpha-1-antitrypsine. (101)
Ces considérations concernent les modes de variations génétiques, dans les portions codantes du génome, soit environ 5% de l'ADN génomique total.

A propos des 95% restant, des estimations évaluent la différence moyenne entre deux génomes humains à 1/100 ou 1/500 nucléotides.

La comparaison pour un individu donné, de la séquence nucléotidique d'origine paternelle, à celle d'origine maternelle, donnerait plus de 10 millions de différences, correspondant à l'accumulation de mutations au cours de l'évolution humaine.
La proportion totale de bases polymorphiques *(index d'hétérozygotie de l'ADN)* est environ dix fois plus grande que la proportion de nucléotides hétérozygotes estimés pour les régions du génome codant, pour une protéine (1/ 2500 paires de bases pour 1/270 paires de bases polymorphiques).

Il semble en effet plus probable que les régions codant pour les protéines, soient soumises à une pression sélective beaucoup plus rigide que les régions non codantes, ce qui expliquerait la présence de mutations plus faible dans ces régions.

On notera l'intérêt de la méthode de Southern (exploration au moyen de sondes d'ADN cloné la variation des sites de restriction dans une région particulière de l'ADN, détection d'un fragment d'ADN génomique pour un million).

### Polymorphismes de la longueur des fragments de restriction (RFLP) (101)

Tous les individus n'ont pas exactement la même distribution des sites de restriction.

Le degré de variation détecté par la technique de Southern a été au-delà de la prédictibilité de la variation au niveau nucléotidique présumée, grâce aux connaissances des mutations et polymorphisme des protéines.

Des changements de séquence dans l'ADN génomique, due à une mutation héritée ou à une néomutation, peuvent conduire à la formation ou à la perte de sites de reconnaissance, ce qui va entraîner des modifications dans la taille d'un ou plusieurs fragments, révélés par l'hybridation avec une sonde donnée, lors d'une analyse en Southern.

Cette méthode permet un examen approfondi de la séquence de nucléotides dans les régions voisines d'une sonde d'ADN, puisque plusieurs centaines d'enzymes de restriction sont disponibles pour détecter une très grande variété de séquences de 4 à 8 paires de bases.

On peut facilement examiner, par cette analyse, la longueur des différents fragments, reflétant le génotype à un site de restriction particulier.

Les RFLP peuvent également apparaître après délétion ou insertion d'un segment d'ADN plutôt que suite à un changement dans un nucléotide. La taille du fragment de restriction en sera d'autant modifiée.

Les polymorphismes de l'ADN représentent simplement la manifestation moléculaire de la variation du génome qui est apparue depuis les études du polymorphisme au niveau des protéines.

### Polymorphismes en série de fragments alléliques de longueurs variables fermés par un nombre variable d'une séquence d'ADN répétée en tandem (VNTR) (101)

Une classe spéciale de polymorphisme par insertion / délétion, consiste en une série de fragments alléliques de longueurs variables, fermés par un nombre variable d'une séquence d'ADN répétée en tandem et située dans l'intervalle entre deux sites de restriction.

Cette classe RFLP est qualifiée d' "hypervariable".

Ces loci se caractérisent par un grand nombre d'allèles (plusieurs douzaines, voire plus), de sorte que deux individus non apparentés partagent très rarement les mêmes allèles.

Les marqueurs VNTR sont très informatifs pour les analyses de liaisons génétiques.

Les RFLP bi-alléliques et les VNTR ont augmenté le nombre de marqueurs polymorphes permettant d'examiner la diversité génétique existant entre les individus, et de suivre la ségrégation de différentes portions du génome dans les familles. Plusieurs milliers de ces polymorphismes d'ADN localisés sur l'ensemble des chromosomes ont maintenant été décrits. (101)

### 2-5 TNF-α, interactions avec l'écosystème

Le gène du TNF α humain est localisé sur le chromosome 6, dans une région entre p23 et q12, proche de l'ensemble des gènes du complexe majeur d'histocompatibilité (CMH). La protéine est codée par un gène unique de 3,6 kb, comportant trois introns et quatre exons.

Le TNF α est une protéine transmembranaire de type II de 233 acides aminés, avec une extrémité C-terminale externe à la cellule et un domaine cytoplasmique.

Cette protéine peut être clivée par une métalloprotéase membranaire, appelée TNF convertase enzyme (TACE).

Le TNF-α ainsi libéré (17kD), peut s'assembler en trimère circulant. L'extrémité C-terminale de chaque sous-unité est incorporée dans la base du trimère, l'extrémité N-terminale restant relativement libre.

Ainsi, le TNF-α peut exercer sa fonction, soit à distance par la forme soluble, soit lors de contact intercellulaire par la forme membranaire.
La structure tridimensionnelle du TNF-α a été résolue et décrite à haute résolution. (7)

L'expression du gène du TNF-α est stimulée par une large variété d'agents, essentiellement des facteurs bactériens et des stimuli immunologiques.

Concernant les macrophages, l'expression du TNF-α est induite par des stimuli biologiques, chimiques et physiques, tels que des produits issus de virus, bactéries ou parasites, des cellules tumorales, le complément, des cytokines (IL1, IL2, INFγ, GM-CSF, M-CSF, et TNF-α lui-même), l'ischémie, le trauma, les irradiations.

Pour les autres types cellulaires, d'autres stimuli peuvent être efficaces: le LPS pour les monocytes, les rayonnements ultraviolets pour les fibroblastes, les esters de phorbol et infections virales pour de nombreux autres types cellulaires. (7)

### 2-6- Les parodontites associées aux maladies à transmission mendélienne. Implications génétiques et héréditaires.

L'approche épidémiologique de l'étiologie des maladies parodontales confirme leur caractère multifactoriel, dont les caractéristiques microbiennes, environnementales, systémiques ou génétiques constituent autant de facteurs modulant leur expression.

Il est clairement établi aujourd'hui que les individus ne sont pas égaux devant le risque de développer une maladie parodontale.

Chez les patients à haut risque, les facteurs de susceptibilité de l'hôte, génétiquement déterminés, jouent un rôle significatif dans la manifestation de cette maladie. (38)

Ces données ont pu être récoltées à partir d'études concernant la manifestation de formes agressives de la maladie au sein de mêmes familles.

Les résultats d'étude d'expression chronique de la maladie parodontale chez des jumeaux, en désignent une composante génétique significative.

Les symptômes accompagnent aussi des maladies systémiques transmises selon un mode mendélien. L'association de ces parodontites sévères à ces modalités de transmission mendéliennes présente plusieurs implications importantes:
■ La susceptibilité à la maladie parodontale associée au syndrome est transmise héréditairement
■ La localisation génétique du locus de la maladie permet de poser les bases biochimiques ou génétiques de tests diagnostics.
■ L'avancée de nos connaissances sur ces maladies permettra de définir les causes plus précises du déclenchement des parodontites associées. (38)

(38) Il a été par ailleurs établi (9) que les antigènes de type HLA-DR4 doivent être considérés comme des facteurs de risque pour "la parodontite à progression rapide", en particulier pour certains de ses sous-types (0401, 0414,0405 et 0408). Il est à remarquer que ces déterminants ont été incriminés dans la polyarthrite rhumatoïde.

Certaines informations montrent que des formes précoces de maladies parodontales (EOP: early-onset periodontal diseases), actuellement regroupées sous le nom de parodontites agressives, suivent une loi de transmission mendélienne d'un gène à effet majeur.

Le génotype ainsi transmis peut prédisposer des individus à une parodontite quand ils sont contaminés par certaines bactéries. (87')

Le dilemme apparent entre la susceptibilité génétique de réponse à une bactérie spécifique et l'altération de la réponse immune à une bactérie présente normalement dans la flore, pourrait peut-être levée par l'analyse de la cascade réactionnelle, due au fonctionnement du TNF-α.

Le promoteur du TNF-α possède des sites de fixation pour différents facteurs de transcription, principalement NF-κB et AP-1 mais aussi AP-2, NF-AT (nuclear factor of activated T cell), CRE (cyclic adenosine monophosphate response element).

Ces différents facteurs de transcription peuvent varier et interagir de façon différente, selon le type de stimulus et le type de cellule stimulée.

Le polymorphisme du promoteur du TNF-α influence les capacités individuelles de synthèse du TNF, et donc potentiellement la susceptibilité aux maladies inflammatoires (production élevée de TNF) et aux risques d'infections (faible production de TNF). (4)

La transduction du signal fait suite à la fixation du stimulus (physique : UV, RX, chaleur), chimique, immunologique, infectieux sur son récepteur membranaire.

Cette activation agit sur NF-Kb, qui, à l'état basal, est séquestré dans le cytoplasme des cellules non activées par les molécules inhibitrices de la famille IκB.

L'activation cellulaire provoque une phosphorylation de l'inhibiteur IκB par un complexe Iκκ.

IκB ainsi phosphorylé, libère NF-κB, puis est dégradé par le protéasome. NF-κB libre transloque alors vers le noyau où il exerce son rôle d'activateur de la transcription.

Dans ce cas, la liaison du stimulus à son récepteur induit le recrutement de protéines intracellulaires (TRAF, TRADD, et RIP) qui induisent une cascade de phosphorylation via JNK et P38.

Les MAPK aboutissent ensuite à l'activation du facteur de transcription AP-1.

Le TNF-α est produit à des niveaux indétectables dans les cellules quiescentes, mais dans les macrophages activés, le TNF-α est une des principales protéines sécrétées. Le gène du TNF-α est un gène de la réponse immédiate.

### 2-7- Facteurs exogènes

### 2-7-1- Alimentation, stress et équilibre de vie

L'alimentation équilibrée constitue un élément à prendre en considération, en dehors des évidences connues, dans l'équilibre biologique de l'individu.

La complémentation alimentaire en antioxydants est considérée aujourd'hui comme un facteur limitant l'action des radicaux libres (évoquée plus loin).

Les polymorphonucléaires produisent des oxydants des protéinases et autres facteurs de destruction, en réponse aux bactéries pathogènes.

L'équilibre entre ces facteurs, les antioxydants et les antiprotéases synthétisées dans l'organisme du patient détermine l'étendue des lésions parodontales. La malnutrition (PEM: malnutrition protéino-énergétique) est caractérisée par une déplétion tissulaire marquée, concernant des substances nutritives antioxydantes clés, GSH comprise, et une altération de la réponse protéinique de la phase aigue (APR) aux infections.

Cette dernière détermine une diminution de la production des protéines de la phase aigue (APP).

L'APR joue un rôle décisif pour favoriser la cicatrisation. Son déficit, dans la PEM, est dû à l'altération de la production des cytokines et de leur action cellulaire.

### 2-7-2-Tabagisme

Le tabagisme est considéré aujourd'hui comme le, sinon l'un des facteurs de risque majeur dans la progression de la maladie parodontale. Des modèles ont été proposés pour illustrer la réponse parodontale de l'hôte vis-à-vis d'une activité microbienne pathogène. (83)

L'influence de la consommation de tabac s'exprime d'une part, au niveau de la cavité buccale et des tissus parodontaux et, d'autre part, sur la flore.

Chez le fumeur, au moment de la consommation de tabac, les concentrations faibles "chroniques" des produits tabagiques relevés dans le sérum, la salive, le fluide gingival, et même les cellules et la matrice extracellulaire des tissus parodontaux sont multipliés par plusieurs centaines, voire milliers, sachant que la fumée tabagique contient entre deux à trois mille substances toxiques ( nicotine, nitrosamines, benzènes, aldéhydes, monoxyde de carbone..,).

Chaque composant a capacité à modifier la réponse de l'hôte, certains d'entre eux cumulant des effets positifs et négatifs.

La nicotine, par exemple, stimule la motilité des neutrophiles et réduit l'adhésion des fibroblastes.

L'influence du tabac sur le développement et la distribution des bactéries pathogènes est controversée.

Des études récentes font état d'une recrudescence d'organismes surinfectants comme E. coli ou Candida.

Dans le volume de la poche, les réactions de défense font intervenir les neutrophiles, secondés par le système du complément et les anticorps, pour débarrasser le biofilm des pathogènes et leurs toxines.

En fait, ces mêmes neutrophiles relarguent, dans le milieu des protéases et médiateurs de l'inflammation, qui contribuent à détruire le tissu parodontal.

Il est reconnu que la consommation de cigarettes conduit à réduire les pouvoirs protecteurs des neutrophiles et anticorps et accentuent les destructions tissulaires.

Des études ont démontré une réduction des IgG et IgA, une réduction des capacités de phagocytose et chimiotactisme des neutrophiles exposés à des niveaux élevés de fumée tabagique.

La fumée stimule l'expression de l'adhésion par les intégrines et inhibe l'expression des sélectines, augmente le nombre de cellules T qui utilisent ce même système de migration tissulaire.

De nombreuses études ont montré une augmentation de concentration des enzymes destructrices telles que βglucuronidase, élastase, et métalloprotéinase-8.

Après que les toxines microbiennes aient infiltré les tissus mous, interviennent les monocytes, avec leurs cytokines inflammatoires et destructrices (IL1-α, IL1-β, TNF-α, PGE, IL-6 et/ou IL-8). D'effet inverse, les médiateurs: TGF-β (transforming growth factor-β), IGF (insulin like growth factor), interférons.

La proportion relative de telles ou telles molécules déclenche une action respectivement destructrice ou réparatrice.

En règle générale, les études les plus récentes font état d'une réduction des cytokines à valeur réparatrice (IL-4) et augmentation de celles destructrices (IL-6, IL-8, TNF-α) chez les fumeurs, comparés à une population de non fumeurs.

Ces niveaux élevés chez les fumeurs feraient état de la désorganisation des systèmes de défense des tissus parodontaux.

D'autres facteurs, comme la génétique, l'alcool et le stress psychologique, interagissent avec le tabac, pour amplifier les voies inflammatoires destructrices, au cours des maladies parodontales.

Ces dernières années, le rôle majeur des polymorphismes d'allèles de gènes codant pour l'IL-1, le TNF-α, l'IL-6 et le récepteur FC-γ ,entre autres, a été démontré, définissant une prédisposition de certaines populations à développer et/ou aggraver la maladie parodontale.

La conséquence en est une amplification de la sécrétion/activité des médiateurs destructeurs de l'inflammation et/ou une réduction du niveau/activité des éléments protecteurs de la réponse de l'hôte.

Il a été démontré par exemple que le polymorphisme de l'allèle 1 G de la métalloprotéinase, associé au tabagisme, se rencontre fréquemment dans les cas de perte dentaire après traitement parodontal.

De même, avec le génotype Fcγ-IIa chez les fumeurs, dans une population chinoise atteinte de parodontite agressive généralisée.

Une mutation doublement allélique du gène codant IL-1, chez des fumeurs a accru le risque de déclenchement de maladie parodontale, par opposition à l'échantillon non fumeur.

Plus récemment, les polymorphismes des enzymes cytochrome 450 et glutathioneS-transférase (réputées neutralisant les substances tabagiques et inflammatoires), mettent les fumeurs en plus grand risque de développer une maladie parodontale.

Enfin, il semblerait que les tétracyclines neutralisent les médiateurs de l'inflammation particulièrement accrus chez les patients fumeurs atteints de maladie parodontale, notamment réprimant plusieurs métalloprotéases (y inclus les collagénases) et inhibant le relarguage de IL-1β des monocytes.

Il semblerait que la prescription de doxycycline, par voie systémique, ou bien de minocycline et doxycycline, par système à émission contrôlée, réduisent les effets délétères du tabac, associée au curetage et surfaçage radiculaire.

Il a été évoqué récemment, que la délivrance locale de tétracyclines n'exerce pas seulement un effet antimicrobien, mais contribue aussi à un effet modulateur chez l'hôte, en le protégeant lui-même des effets amplifiant du tabagisme sur les aspects destructeurs de l'inflammation.

Il a été rapporté par ailleurs(11), que le taux plasmatique en antioxydants en général, (vitamine C, vitamine E, carotènes), est réduite chez le fumeur, tant par une plus faible tendance à la consommation que le résultat d'une plus faible absorption et augmentation de sa destruction.

Fumer une seule cigarette réduit la concentration salivaire et plasmatique en GSH. (11)

Les polymorphonucléaires circulant contiennent plus de superoxyde chez le fumeur. D'autre part, le taux de GSH dans le ligament desmodontal est relativement plus faible en cas de tabagisme.

Enfin, il a été démontré que la GSH a un effet protecteur contre la cytotoxicité de la nicotine sur les fibroblastes du ligament parodontal. (11)

Même si le tabagisme est reconnu être un facteur de risque aggravant vis-à-vis de la maladie parodontale, l'étude de la production par les neutrophiles en ROS est relativement récente.

### 2-7-3- Stress oxydatif

### Introduction:

Le principe fondamental à la compréhension de cette approche est le suivant:
Les moindres changements, aussi subtiles soient-ils, de l'état d'oxydoréduction intracellulaire concernent les évènements de transcription génique qui risquent de provoquer des dommages tissulaires consécutifs au déclenchement d'un état pro-inflammatoire. (11)

### 2-7-3-1.Principes généraux. Données scientifiques.

Les radicaux libres sont des éléments capables d'autonomie contenant un ou plusieurs électrons singulets.

Ils sont par nature hautement réactifs et capables d'extraire des électrons et de fait d'oxyder nombre de molécules biologiques. Ils regroupent non seulement les radicaux libres oxygénés mais aussi à nitrogène et chlorure (par exemple, le peroxynitrite ONOO-, produit de la réaction : NO˙+ O²- ---> ONOO-).

Ce produit a fait l'objet de développements notamment pour signaler sa profonde sous-estimation dans la pathogénèse des maladies inflammatoires. (11)

Le terme **ROS** (reactive oxygen species: éléments à oxygène actif) est devenu plus familier. Il englobe aussi d'autres éléments réactifs qui ne sont pas de vrais radicaux libres mais sont capables de contribuer à la formation de radicaux libres à l'intérieur des cellules ou l'environnement extracellulaire.

Le terme **AO** (antioxydant species: antioxydants) désigne les substances qui, à faible concentration relativement à un substrat oxydable, retarde ou empêche significativement l'oxydation de ce substrat.

Un dérèglement du **ratio radicaux libres/ anti-oxydants** provoque des dommages immédiats intracellulaires au niveau des structures et des molécules biologiques vitales, de la membrane cellulaire, jusqu'à la nécrose ou une apoptose accélérée. Les dommages extracellulaires concernent les tissus conjonctifs minéralisés ou pas, la matrice extracellulaire et ses composants.

Les systèmes de défense anti-oxydants sont désormais désignés avec beaucoup de sérieux pour leur capacité à restituer l'équilibre redox intracellulaire et leurs influences sur la transcription génique et les vecteurs d'information cellulaire. A été évoquée aussi leur faculté à prévenir la formation d'éléments réactifs, les éliminer et réparer leurs dommages.

Le **stress oxydatif** a été défini par SIES, comme une perturbation de l'équilibre ROS/AO, en faveur du premier, conduisant à un dommage potentiel.

Un contexte physiologique se définit par un équilibre dynamique entre l'activité ROS et la capacité de défense AO.

Le stress oxydatif résulte d'une réduction de l'activité de défense AO ou une augmentation de la production ou de l'activité de ROS.

Fort de cela, il a été estimé une production cellulaire quotidienne de 1 à 3 milliards de radicaux libres... ce qui désigne les systèmes de défense antioxydants comme éléments primordiaux de la préservation de l'équilibre de santé.

Le **potentiel redox** (volt) est une mesure d'affinité d'une substance pour les électrons, relative à l'hydrogène, affinité 0.

Les substances plus électronégative que l'hydrogène ont un potentiel redox positif et sont des **oxydants;** celles moins électronégatives ont un potentiel redox négatif et sont des **réducteurs.**

Dans le sillon sulculaire ou la poche parodontale, *un potentiel redox faible* est considéré comme *essentiel à la croissance et la survie des anaérobies sous-gingivales* mais aussi *un environnement réducteur protège du stress oxydatif et de ses effets*.

Il en résulte une contradiction apparente dans la stratégie de traitement des maladies parodontales qui voudrait préserver un potentiel redox bas pour favoriser la réparation tissulaire mais qui, en contre-partie, encouragerait la croissance et la survie de la flore pathogène...

Quoi qu'il en soit, rappelons que l'organisme est compartimenté. Un état redox bas intracellulaire ne présume pas de l'état redox du volume sous-gingival. (11)

Cette affirmation pourrait susciter des questions (lire plus haut 1.3.1). L'internalisation de P. gingivalis apte à se reproduire dans le milieu endocellulaire serait elle à même d'influencer le potentiel redox?

Cette compartimentation de l'organisme est étayée par des études dont l'exemple type est l'analyse des proportions relatives dans le fluide gingival d'une part et le plasma et la salive d'autre part en glutathion réduit(GSH) et en acide urique.

Le premier prédomine dans le fluide gingival et le second dans salive et plasma où on mesure aussi une forte concentration de complexes protéines-antioxydants. (11)

Les radicaux libres ont une *source exogène* (fièvre, traumatisme, ultrasons, rayons ultraviolets, ozone, tabagisme, fumées exogènes, radiations, infection, surentraînement sportif, drogues médicamenteuses) et une *source endogène* (produits des voies métaboliques et le système mitochondrial de transport d'électron formant le superoxyde, la fabrication fonctionnelle par les cellules de défense de l'hôte (phagocytes) et les cellules conjonctives (ostéo- et fibroblastes).

Des articles richement documentés décrivent en détail les processus réactionnels faisant intervenir les radicaux libres dans le métabolisme cellulaire (11), consommant l'oxygène y compris pour la glycolyse intramitochondrial, le cycle des acides aminés et la formation d'ATP.

La combustion incomplète de l'oxygène métabolique est estimée à un pourcentage de 1 à 3% de l'oxygène consommé au- delà de la capacité mitochondriale antioxydante à dégrader le superoxyde.

La superoxyde dismutase 2 -Mg dépendante, dégrade le superoxyde formé.

Quoi qu'il en soit, les dommages causés par les ROS et les éléments nitrogènes réactants (RNS) sur l'ADN mitochondrial engagent un processus considéré comme important dans certaines maladies chroniques et le vieillissement.

Ce processus, comprenant la combustion respiratoire à l'intérieur des PMN neutrophiles, est catalysé par de nombreux mitogènes/cytokines et d'autres médiateurs tel que le granulocyte-macrophage colony-stimulating factor.

L'organisme réagit par les systèmes de défense **antioxydants** répertoriés selon leur *modes de fonction* ou *de localisation,* leur *solubilité, la structure support* ou leur *origine.*

### 2-7-3-2. Evaluations qualitative et quantitative

### L'acide ascorbique ( vitamine C)

Ses actions au cours de la maladie inflammatoire se résument en:
■ neutralisation des radicaux peroxyl hydrosolubles
■ neutralisation des radicaux superoxyde et perhydroxyl
■ prévention des dommages occasionnés par les radicaux hydroxyl sur l'acide urique
■ neutralisation de l'hypochlorite
■ réduction de la dégradation de l'hémoglobine et le relarguage de l'ion ferreux Fe²+, prévenant ainsi les réactions de FENTON
■ neutralisation de l'oxygène singlet et des radicaux hydroxyl
■ recomposition de l'α tocopherol (vitamine E) depuis son radical
■ protection des dépôts de radicaux libres dus à la fumée tabagique

Plus récemment, a été mis en évidence sa capacité à réduire la C-réactive-protéine- mediated, molécule de l'adhésion monocytaire.

La concentration de la vitamine C est dans le fluide gingival, trois fois celle du plasma. Il a été démontré qu'elle prévient l'action de la collagénase des neutrophiles.

L'ascorbate peut être réduit en radical ascorbyl par attaque oxydative, donnant le déhydroascorbate qui inhibe la vitamine.

Cette action oxydative est réverse par action de la GSH réduite ou la NAD-semi-dehydroascorbate réductase, système enzymatique utilisant aussi la glutathion (GSH).

Toutes ces actions sont endocellulaires. L'ascorbate extracellulaire est rapidement oxydé dans des conditions de stress oxydatif, tant que la GSH n'atteint pas une concentration extracellulaire adéquate (1-2 µM).

Mentionnons d'autres antioxydants:

### ■ α-tocophérol( vitamine E)

Considéré comme l'antioxydant liposoluble le plus actif in vivo, majeur pour préserver l'intégrité membranaire contre la peroxydation lipidique en neutralisant le radical peroxyl.

Ses actions sont:
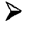 inhibition de la protéine-kinase C et l'agrégation plaquettaire
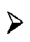 inhibition de l'oxyde nitrique par l'endothelium vasculaire
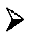 inhibition de la production de superoxyde par macrophages et neutrophiles par phosphorylation de p47phox, pendant l'activation de la NADPH oxydase

Les limites de son efficacité antioxydante tiennent à sa faible mobilité à travers la membrane cellulaire, sa carence dans les milieux hydrosolubles du fait de leur richesse en radicaux libres.

### ■ Les caroténoïdes

On en connaît plus de 600 variétés (lycopène, α-carotène, rétinol,..), strictement d'origine alimentaire végétale (tomates, pamplemousse rouge, pastèque,..). Les avis divergent pour considérer la vitamine A en tant que antioxydant puisque son intégrité dépend de la pression interne en oxygène de l'écosystème ambiant.

### ■ Le co-enzyme Q10

Il existe sous forme oxydée (ubiquinone ou CoE Q) et réduite (ubiquinol ou CoE QH2) qui, les deux, ont une activité antioxydante. La carence de cet agent a été démontrée dans certaines maladies neurodégénératives et aussi dans le tissu gingival de sujets atteints de parodontite.

Des études chez le patient à parodontites sont attendues pour évaluer l'intérêt de ce CoE dans les traitements parodontaux.

### ■ L'acide urique

Contenu dans le plasma, l'urine et la salive, c'est un des agents neutralisant les radicaux majeurs dont:
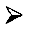 l'oxygène singlet
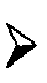 l'hydroxyl
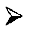 l'acide hypochloride

Il protège la α1- antitrypsine combinée à l'ascorbate et se combine aux cations métalliques bivalents en prévenant les réactions de FENTON. Normalement, il est oxydé en allantoïne, marqueur de l'oxydation de l'urate par les ROS, in vivo.

### ■ Les polyphénols

De nombreux dérivés sont contenus dans les légumes, le vin rouge et le thé.

Ils interviennent dans la protection vitaminique.

### ■ Le glutathion

Tripeptide non essentiel, il peut être synthétisé dans le milieu cellulaire, même si ses acides aminés essentiels ne sont fournis que par voie alimentaire. Ses formes oxydée(GSSG), réduite(GSH) interviennent à plusieurs titres:
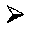 SH est un des antioxydants majeurs intracellulaire
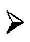 Essentiel pour le système enzymatique glutathion peroxydase, maintenant l'équilibre redox intracellulaire
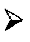 Neurotransmetteur régulant des fonctions neuro-immune -endocrines
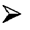 Préserve certaines vitamines(C et E)
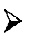 Contrôle la production de cytokines pro- inflammatoires en régulant l'expression et/ou l'activation de facteurs de transcription redox (facteur nucléaire KB, et activing-protein-1)
Son rôle est complexe puisque la synthèse de GSH est régulée par des cytokines. (11)

Les **systèmes de défense antioxydants** sont complexes et physiologiquement intriqués dans la biologie de l'organisme.

Les études les concernant nous apportent des éléments de compréhension, mais ne sont pas reproductibles in vivo, du fait de leur ubiquité extra ou intracellulaire, difficilement dissociable.

Plusieurs tentatives d'évaluation de défense globale antioxydante ou"capacité antioxydante totale" ont été développées pour réduire le coût et le temps passé à l'évaluation individuelle des éléments antioxydants.

Il semblerait que l'effet antioxydant global soit supérieur à la somme des effets antioxydants de chaque élément dépisté. Il s'avère aussi que l'influence de ces éléments soit relativement méconnue et techniquement difficile à cerner.

Néanmoins, il apparaît que :
*Le milieu buccal constitue un écosystème de choix pour explorer les activités des ROS et des antioxydants en composant des modèles biologiques d'évaluation de complémentation topique en ces substrats,* à *des concentrations significatives, dont le but serait de mettre en oeuvre des thérapies innovantes, au-delà des besoins parodontaux.*

Le rôle de la nutrition dans les maladies inflammatoires y compris parodontales a été évoqué. (11, 21')

Il semblerait que les supplémentations alimentaires ne compenseraient pas, pour réduire le stress oxydatif par des éléments pro-oxydants, aussi efficacement que l'alimentation complète à base de concentrés de fruits et légumes, notamment en apport de GSH et vitamine E.

Par ailleurs, il n'existe pas, à ce jour, de moyen reproductible visant à mesurer la capacité antioxydante ni les dommages causés par les radicaux libres.

Les radicaux libres ont une durée de demi-vie très courte *in vivo,* (10-6,10-9 s) et ne peuvent, de ce fait, être mesurés directement. Des systèmes *in vitro,* les"spin traps" ou "piège à spin", ne sont pas applicables in vivo à cause de leur toxicité méconnue.

Ces pièges utilisés sont l'acide ascorbique, des cycles aromatiques (salicylate, phénylalanine,..), l'urate.

Des bio-marqueurs de la peroxydation des lipides, des dommages causés à l'ADN et aux protéines ont été mis au point, pour tracer les dérivés de l'attaque radicale des structures tissulaires.

Les antioxydants se répartissent selon les compartiments de l'organisme, hydro- ou liposolubles et, pour certains, passerelle entre les deux fractions.

Les bactéries de la plaque et leurs produits sont une source avérée de facteurs qui peuvent stimuler les neutrophiles infiltrant les tissus parodontaux.

L'idée, que les radicaux libres puissent être corollés à la pathogénie de maladies inflammatoires, a nourri de nombreuses études ces dix dernières années, sans pour autant emporter le suffrage scientifique.

Les postulats d'HALLIWELL, à l'instar de KOCH en 1884, décrivent les pré-requis des radicaux libres, pour en faire les médiateurs pivots des dommages tissulaires dans une maladie donnée:
*les ROS ou les dommages oxydatifs doivent être présents sur le site de l'altération.*
*La formation ou la dégradation oxydative causée, doivent* pouvoir agir simultanément ou avant la destruction.
(Exemple de cette approche par une réflexion à propos des patients traités de cancers, voir 2-7-5)

### 2-7-4-Vieillissement cellulaire et apoptose

Le vieillissement provoque l'altération structurale et fonctionnelle des tissus conjonctifs.

Ces modifications ont été illustrées par les variations biologiques de la fibronectine, à titre d'exemple.

Ce phénomène a été impliqué dans la destruction des tissus parodontaux. L'activation des caspases 3 et 7 est très augmentée dans les tissus gingivaux atteints de parodontites.

P. gingivalis, en association avec la protéine Sip (immunosuppressive protein) de T. denticola, montre la capacité d'induire l'apoptose chez les lymphocytes T.

P. gingivalis augmente l'expression du Fas ligand et en activant les caspases 3.

L'interaction entre des facteurs de croissance tels que TGF-β (Transforming Growth Factor-β) et chondroitin sulfate ou dermatan sulfate, qui servent de sites de fixation à ce facteur au sein de la matrice extracellulaire, favorisent la croissance et la différenciation cellulaire.

Il apparaît aujourd'hui que les modifications structurales et fonctionnelles de certaines macromolécules, composant la matrice extracellulaire protéoglycanes), affectent significativement le potentiel de régénération tissulaire et engage, de fait, l'organisme dans le processus de vieillissement, en perturbant cette horloge biologique.

B. forsythus, par ses lipoprotéines, induit l'apoptose dans différentes lignées cellulaires humaines.
Ce phénomène provoqué aussi par les bactéries du complexe rouge, contribue à la progression des parodontites, par la réduction des leucocytes dans la poche parodontale et l'altération des tissus.

### 2-7-5-Intercorrélation avec des maladies générales neurodégénératives et tumorales

Cette question apparaît sans relation directe avec le sujet traité mais sert de support à réflexion globale.

### 2-7-5-1. Un exemple, la polyradiculonévrite aigüe ou syndrome de Guillain-Barré

Cette pathologie se caractérise par une inflammation des racines nerveuses périphériques. Il semblerait que des effecteurs immunitaires cellulaires et humoraux interviennent contre des constituants du nerf périphérique. La forme la plus classique est celle d'une polyradiculonévrite inflammatoire aigüe avec démyélinisation segmentaire multifocale. Il existe de nombreuses variantes.

Non liée au sexe, cette maladie est marquée par un antécédent infectieux respiratoire ou digestif dans les 15 jours (70% des cas); plus rarement suit une vaccination. Son évolution neurologique est le plus souvent ascendante, sensitive et/ ou motrice. Le déficit moteur est très variable. Le déficit sensitif, à prédominance proprioceptive avec ataxie possible. Le système nerveux végétatif souvent atteint est source de complications aggravantes. La récupération pas toujours complète ne survient plus au-delà de 12 à 18 mois.

La recherche, à utilité physiopathologique et non diagnostique, de médiateurs de l'inflammation met en évidence notamment une augmentation de cytokines pro-inflammatoires.

Le système nerveux périphérique (SNP) est sensé être séparé des effecteurs du système immunitaire cellulaire et humoral par la barrière hémato-nerveuse (BHN) constituée par les jonctions serrées des cellules endothéliales. Cependant, les lymphocytes activés peuvent traverser la BHN et interagir avec les cellules du SNP.

La première étape de la maladie serait une activation d'effecteurs lymphocytaires pouvant alors adhérer aux cellules endothéliales et pénétrer dans le SNP. Ils induisent ensuite une inflammation, une altération de la BHN, un recrutement inflammatoire cellulaire L( T, NK ,B), macrophages et sérique (AC, cytokines).

Ces médiateurs peuvent être produits par des cellules infilrantes ou des cellules du tissu nerveux lui-même. L'activité de ces substances provoque une démyélinisation segmentaire inflammatoire, inégalement répartie le long des fibres nerveuses. Une lésion sévère peut aller jusqu'à la rupture de l'axone.

Les causes de l'agression immunologique mal connues orientent malgré tout les soupçons vers une origine infectieuse avec mise en cause de Campilobacter jejuni, VIH, le groupe herpes ( cytomegalovirus, herpes virus, MNI), avec mimétisme moléculaire entre parois bactériennes et glycoprotéines et glycolipides du nerf périphérique. Des auto-anticorps ont été mis en évidence, reconnaissant à la fois les parois bactériennes et les constituants du système nerveux. Cette hypothèse n'est pas exclusive. Les agents infectieux peuvent provoquer une immunosuppression. Par l'induction de cytokines, ils peuvent favoriser une manifestation auto immune ou immunoallergique. L'aspect réversible de cette pathologie, même avec séquelle de gravité très variable, attire notre attention sur le caractère inflammatoire surexprimé accompagnant tout le processus plus particulièrement immunitaire spécifique.

La question que sans doute nous pouvons nous poser au travers de cette maladie évoque peut-être des territoires fonctionnels prédisposés à l'expression de ce désordre physiologique concernant l'intimité biologique immunitaire- non spécifique ou spécifique- de l'organisme.

Tel ou tel patient se verrait affecté dans son intégrité neurologique, cellulaire ou parodontale selon ses faiblesses acquises ou innées.

### 2-5-2. Réflexion à propos de thérapies vis-à-vis de maladies tumorales

L'indication et l'efficacité d'une thérapie de soutien de patients ayant reçu un traitement systémique, tel une chimiothérapie ou des thérapies combinées, pour éradiquer des cancers, reste un débat entier.

Il est clair que ces patients, ayant subi les conséquences toxiques très souvent lourdes des drogues administrées, présentent « une réponse clinique objective».
Cette thérapie de soutien ne peut pas être considérée comme une thérapie ultérieure au traitement de fond.

Elle ne vient en aucun cas compenser la toxicité des drogues et/ou la probabilité plus faible d'obtenir une réponse meilleure ou plus étendue au traitement du fait de la résistance acquise des cellules tumorales exposées aux médicaments cytotoxiques. Elle ne donne souvent qu'une réponse de faible durée. En quoi consiste- t- elle?

Malgré ces réserves, l'utilisation de substances non ou peu toxiques et potentiellement actives tels que des hormones et des molécules de l'immunothérapie semblent correspondre à une logique de traitement, malgré la pauvreté bibliographique. (62)

Les patients atteints de cancer à stade avancé présentent une immunodéficience significativement plus marquée étroitement liée à l'altération de la fonction immune à médiation cellulaire IL-2 dépendante, ce, associée à de hauts niveaux de cytokines pro-inflammatoires ( IL-6, TNF-α).

La correction immunologique a aussi pour objectif d'éradiquer les clones de cellules tumorales résistant à la chimiothérapie.

IL-2, facteur de croissance des cellules-T (TCGF), présente une diversité de propriétés immuno- modulatrices:
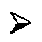 Stimulation de la prolifération des cellules-T
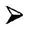 Stimulation de la fabrication des lymphocytes T cytotoxiques
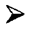 Stimulation de l'activité anti tumorale des cellules tueuses (natural killer NK)

Le mécanisme d'action de IL-2 dépend de son interaction avec son récepteur membranaire spécifique.

La haute affinité IL-2 R est assurée par trois sous-unités, α (CD25), β (CD122), γ (CD132).

La forme complète s'exprime seulement après activation lymphocytaire. CD122 et CD132 sont présents sur les lymphocytes non activés; CD25 n'est présent qu'après leur activation.

De fait, son expression est un marqueur spécifique de l'activation lymphocytaire. CD25 passe à l'état soluble (sIL-2R) et joue un rôle majeur dans le contrôle de la prolifération lymphocytaire. sIL-R peut coapter IL-2 dans le milieu extracellulaire en prévenant la liaison de la cytokine avec son récepteur.

Cette activité cytotoxique primaire de IL-2 est due à l'induction de l'activité de cellules tueuses activées par des lymphokines endogènes (endogenous lymphokine-activated killer cell : LAK). Une injection intraveineuse à haute dose de IL-2 s'est montrée efficace dans le traitement de tumeurs solides mais associée à une toxicité très significative.

IL-2 peut être envisagée comme la cytokine optimale pour réhabiliter la fonction immune à médiation cellulaire, chez les patients cancéreux après chimiothérapie efficace. Mais l'immunothérapie à IL-2 doit rester praticable et bien tolérée. Ce, par des administrations sous-cutanées à faibles doses, réputées actives et dénuées d'effets secondaires.

D'autres études cliniques et expérimentales ont démontré la capacité de l'acétate de megestrol (MA) et de l'acétate de médroxyprogestérone (MPA) à réduire la production de cytokine et à atténuer le syndrome d'anorexie et cachexie (CACS) associé chez ces patients, réduisant les indices de niveau de performance (performance status:PS) et de la qualité de vie (quality of life : QL). MA et MPA sont efficaces et bien tolérés pour traiter le CACS.

Le stress oxydatif a été pris en compte dans cette étude. Les éléments à oxygène actif (reactive oxygen species: ROS) sont normalement décelables dans l'organisme, sous forme des catabolites oxydatifs. Cependant, ils peuvent provoquer potentiellement des dommages cellulaires en oxydant l'acide nucléique, les protéines et les lipides membranaires, dès lors qu'ils atteignent un niveau élevé de production dans des conditions physiopathologiques. Ce sont, nous l'avons vu, les radicaux superoxydes, hydroxyls, peroxyls, l'acide hypochlorique et l'oxygène singlet. Les ROS sont impliqués dans les fontes tissulaires, le CACS et perte de poids.

D'autres cytokines (TNFα) provoquent aussi le CACS, induisent la formation de ROS:symptomatologie classique qui aboutit souvent à une altération de la fonction cellulaire voire une destruction tissulaire. Le stress oxydatif, une des causes de CACS, est potentialisé par un excès de ROS et de cytokines pro-inflammatoires ( IL-1, IL-6, TNF-α).
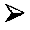 L'acide α lipoïque (ALA) est reconnu réduire les désordres du stress oxydatif ; augmente fortement la forme réduite du glutathion intracellulaire (GSH) ; compense les cellules à forte déficience en glutathion.
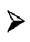 La N-acetyl cystéine (NAC), un autre précurseur de la synthèse de glutathion, agit sur la balance redox en stimulant le potentiel antioxydant en augmentant les niveaux de GSH.

Il est à noter que cette étude s'appuie sur un groupe de patients médicalement sélectionnés de façon drastique et a obtenu l'approbation d'un comité d'éthique universitaire, chaque patient ayant signé un consentement éclairé.

A l'issue de cette immunothérapie, les patients survivants à leur cancer traité par chimiothérapie présentent une augmentation significative du comptage absolu des lymphocytes, IL-2, leptine, GP_{X} et une réduction significative de IL-6, IL-1β, CRP, fibrinogène et ROS. Les patients décédés à l'échéance de l'immunothérapie présentaient seulement un accroissement significatif du comptage absolu des lymphocytes, IL-2 et GP_{X}; une réduction significative des ROS. Cette étude a montré une amélioration de la longévité et de la qualité de vie des patients ayant surmonté la pathologie traitée.

L'objectif majeur de cette approche était l'évaluation des modifications de comptage des lymphocytes pendant le traitement à l'IL-2, connaissant le rôle central des lymphocytes dans la destruction des cellules tumorales. Ce comportement lymphocytaire constitue un marqueur caractéristique de l'activation immune en réponse à l'administration de IL-2. Dans les cas de métastases de cancer du rein, une augmentation plus importante des lymphocytes a été relevée après injection de IL-2.

Au même titre qu'il est connu qu'une lymphocytopénie et /ou un faible pourcentage est de mauvais pronostic pour les patients atteints de cancer (a contrario, inversement) nous savons aujourd'hui d'après une étude récente ( 62) que, en réponse à une immunothérapie à IL-2 , l'augmentation du nombre des lymphocytes va de paire avec une normalisation des valeurs de IL-6 *avant* la mise en place de l'immunothérapie. Si les patients décédés présentaient, avant le soutien immunitaire, un comptage significativement plus faible de lymphocytes comparés aux patients ayant survécu, les valeurs de IL-1β, IL-6, CRP, fibrinogène n'ont pas changé pendant le traitement de soutien. Ce constat peut servir aussi de facteur prédictionnel.

Pour les patients à un stade de cancer avancé, il a été observé un niveau sérique élevé de cytokines ( IL-1, IL-6, TNF-α). MA dérégule la synthèse et le relargage des cytokines et réduit les symptômes du CACS dont la mise en place est lié étroitement à l'activité des cytokines elles-mêmes. MPA réduit in vitro la production de cytokines et de la sérotonine(5-HT) par les monocytes(PBMCs) du sang périphériques de ces patients.

Les paramètres du stress oxydatif sont significativement améliorés après traitement, sans qu'aucune extrapolation puisse être tirée sur leur rôle dans les caractéristiques cliniques des patients.

L'utilité d'un tel protocole est débattue pour les patients présentant un tableau clinique stabilisé après chimiothérapie ou traitement combiné. Pour certains patients non stabilisés, prolonger la chimiothérapie n'est sans doute pas la meilleure réponse apportée, du fait de sa haute toxicité et un ratio avantages /inconvénients faible. Dans ces cas, la biothérapie par IL-2 et facteur stimulant les colonies granulocytes/macrophages devient prévalente.

Ces différents médiateurs du comportement immunitaire sont mis en oeuvre dans des protocoles adaptés aux caractéristiques des cancers rencontrés et leur stade de gravité.

Depuis l'aménagement de IL-2 dans l'immunothérapie anticancéreuse, il a été démontré que l'activité et l'efficacité de cette cytokine ne dépend pas seulement des caractères de la tumeur mais aussi de l'état général de l'hôte et de son bilan immunitaire avant le début de cette thérapie.

Il a aussi été démontré qu'un très haut niveau inflammatoire objectivé par des valeurs anormalement élevées de cytokines pro-inflammatoires et CRP, provoquait une résistance de la tumeur à l'IL-2 thérapie, d'où l'intérêt d'introduire dans le protocole la MPA, reconnue réduire les valeurs de TNF-α, cytokine pro-inflammatoire.(62)

L'augmentation de IL-2 commune à tous les patients correspond bien à une augmentation de synthèse de IL-2. Le rIL-2 a été inclus dans le protocole pour réactiver la fonction immunitaire des patients à stade avancé, d'autant que sa durée de demi-vie est très brève (quelques minutes).

Toutes les réserves ont été émises en conclusion de cette communication non randomisée qui ne présente pas un traitement alternatif ni même un moyen de contrôle, puisque, de surcroît, à ce jour, aucun traitement de ce stade post-thérapeutique conventionnel n'existe.

Pour une très faible toxicité, cette approche thérapeutique permet d'accompagner une stabilisation biologique de patients à l'immunité affaiblie par une chimiothérapie, avec une évolution des marqueurs biologiques prévisibles. La projection future des suites à cette étude ouvre la question de la maintenance thérapeutique en choisissant des marqueurs biologiques encore mieux adaptés, garants d'une réponse durable et améliorée du système immunitaire de l'hôte.

Qu'en est-il de l'approche parodontale?

Le protocole mis en oeuvre (62) présente pour le Parodontiste une intéressante base de réflexion pour envisager peut- être une approche similaire, adaptée selon la proposition suivante. Cette analyse permettrait sans doute de mieux cerner la personnalité biologique à laquelle on destine les soins parodontaux spécialisés.
i. Equilibre staturo-pondéral- régime alimentaire
ii. Sédentarité- tabagisme
*iii.* Biomarqueurs biologiques *(évaluation avant le début du traitement et 6, 12, 24 mois de iii à vii)*
   i. Bactériogramme large, au -delà des complexes orange et rouge
   ii. Test génétique d'évaluation allélique codant pour l'IL-1β
   iii. Comptage absolu des lymphocytes
   iv. Niveaux sériques des cytokines pro-inflammatoires (IL-1β, IL-6 et TNF-α)
   v. Niveaux sériques de IL-2 et leptine, CRP, fibrinogène.
   vi. Niveaux sériques des ROS
   vii. Glutathione peroxydase (GP_{X}), superoxyde dismutase (SOD)

Ces données pourraient sans doute servir de base de recherche pour élaborer un protocole conduisant à une définition fonctionnelle plus fine de la personne biologique confrontée à une forme agressive de parodontite délicate à appréhender selon les moyens thérapeutiques conventionnels.

Une autre étude (12) a établi une corrélation entre le polymorphisme génétique de l'IL-1⁻⁸⁸⁹ et l'accroissement du risque de développer une maladie neurodégénérative (Multiple System Atrophy :MSA) dont la symptomatologie clinique est une combinaison variable des caractères parkinsoniens d'atteinte cérébelleuse, autonomique et pyramidale. Hors mis les facteurs étiologiques génétiques et environnementaux, aucun facteur n'a été identifié, qui contribuerait à déclencher cette affection.

En plus des inclusions cytoplasmiques gliales, une perte neuronale facultative et une microglie activée caractérise MSA. L'activation gliale semble être un signe marqueur précoce et sensible des dommages cérébraux. A ce jour, la question du caractère protecteur ou cytotoxique de cette activation dans la neurodégénérescence reste posée.

D'autres études ont rapporté une inflammation significative à prolifération microgliale arborescente accompagnant des pathologies comme la maladie de Parkinson (PD) ou la maladie d'Alzheimer (AD).

Dans ce dernier cas, l'activation des cellules gliales sont visualisables in vivo par émission tomographique en utilisant un ligand spécifique pour certains récepteurs (sites périphériques liant la benzodiazépine).

La microglie activée synthétise plusieurs cytokines pro-inflammatoires (IL-1α, IL-1β, IL-6, TNF-α) dépistées à un niveau élevé dans le cerveau de AD. D'autre part, la microglie activée est entourée de plaques de β-amyloïde, qui, dans des cultures cellulaires, favorise le relargage de cytokines des cellules gliales. Récemment, les polymorphismes codant pour l'IL-1α et l'IL-1β, ont été désigné comme facteurs d'accroissement du risque de AD, probablement en amplifiant les manifestations inflammatoires dans le cerveau. Des études ont montré que l'allèle 2 amplifié du gène, positionné en locus -889, codant pour IL-1α, est associé à AD.

Le résultat de l'étude mentionnée initialement (12) conclut que le risque de développer MSA est *dose-dépendant* de la forme amplifiée allèle 2 du gène codant IL-1α: 3 fois plus élevé avec des patients de génotype doublement amplifié (2/2) face à des patients hétérozygotes (1/2).

Un effet similaire a été rapporté pour des génotypes amplifiés IL-1α (1/2) et (2/2) pour le risque de développer AD, avec un facteur supérieur à 2.

Le polymorphisme de la région promotrice du gène codant pour IL-1 α⁻⁸⁸⁹ est reconnu être porteur de perturbations fonctionnelles. En fait, l'allèle 2 compose des conditions de compatibilité pour un nouveau site de liaison facteur de transcription, Skn-1, et de fait augmente l'activité transcriptionnelle du gène IL-1α, conformément à l'allèle 1 : le génotype 2/2 induit un surcroît d'activité, comparativement aux génotypes 1/1 et 1/2, respectivement d'un facteur 5 et 2 . Les polymorphismes des gènes de régulation codant pour la cytokine sont reconnus contribuer à leur production et être impliqués dans la susceptibilité individuelle à l'expression inflammatoire. (12)

### 3. LA VOIE 3, INVENTION

Les structures parodontales répondent à une agression au même titre que tout tissu conjonctif. Cependant, leur réponse -le processus inflammatoire-dépend de la nature, la fréquence ou l'intensité des facteurs causaux.
Le diagnostic d'une maladie parodontale devrait inclure, idéalement, non seulement la symptomatologie, mais encore le caractère évolutif avéré ou potentiel qui la caractérisent. (87')

### 3.1 La dent et son parodonte: De l'unité fonctionnelle à l'unité diagnostique

Le problème majeur rencontré en Parodontie est le caractère labile des organes dentaires, de par la dimension finie de l'ancrage parodontal.Dans les cas sévères et/ou évolutifs, le clinicien a dans l'urgence de définir un protocole efficace qui stoppera, dans un premier temps, la progression de la maladie, avant de stabiliser, voire reconstruire, si possible, le parodonte. Le degré de difficulté dépend d'au moins deux critères:
- le nombre de facteurs étiologiques directs ou indirects, locaux généraux ou périphériques.
- le caractère aigu et/ou agressif de la maladie.

On désignera par *unité diagnostique,* l'ensemble fonctionnel dent-parodonte, affublée des caractères étiologiques concernés.

### 3-2- Les tests biologiques. Utilités, critères de choix, qualités et carences.

Les tests biologiques utiles en Parodontie, en matière de diagnostic et pronostic, concernent la flore et la réponse de l'hôte. Ils sont classiquement définis comme des tests pronostics. Cependant, le complément d'information qu'ils apportent permet dans certains cas de préciser le diagnostic établi sur des bases habituellement cliniques.

Ces tests sont donc pour les premiers des tests bactériens et pour les seconds un test génétique d'évaluation de la prédisposition des sujets testés au développement d'une maladie parodontale à expression inflammatoire.

### 3.2.1. Les tests bactériens

Contrairement à la plupart des maladies infectieuses qui sont des mono infections, la maladie parodontale se présente sous une forme plus complexe du fait des associations microbiennes. Il s'avère donc très utile d'identifier les souches pathogènes pour prévoir la médication antibactérienne, accompagnant le traitement, la plus appropriée possible.

Actuellement, *les sondes à acides nucléiques* basées sur la reconnaissance de fragments spécifiques de l'ARN ou de l'ADN de la bactérie permettent de détecter un minimum de 10² bactéries vivantes ou non.

Chaque sonde est spécifique d'un génome bactérien, ce qui représente une limitation de la technique même si la facilité de mise en oeuvre en fait un moyen d'investigation de choix: dépister les pathogènes, même mortes, qui s'expriment au niveau du parodonte seulement si l'hôte est permissif, de par son comportement inflammatoire et/ ou immunitaire. II existe dans cette rubrique aussi le système de sonde PCR (polymerase chain reaction) qui permet de détecter rapidement un grand nombre d'espèces bactériennes, même en faible quantité. Cette technique ne donne cependant pas la sensibilité aux antibiotiques des souches détectées.

*Les anticorps polyclonaux* dirigés contre les anticorps spécifiques d'une espèce bactérienne sont aussi un moyen d'identification. Le test ELISA (Enzyme-like-immunosorbent Assay) visualise la réaction sous forme colorimétrique.

Enfin, *l'activité enzymatique* est un autre moyen d'identification microbienne. Certains pathogènes produisent une enzyme Trypsin-like capable de dégrader les dérivés de la β Naphtilamide comme le N- α- benzoyl-DL-arginine-2-naphtilamide (BANA). Parmi ces bactéries, P. gingivalis, T. denticola, Capnocytophaga, B. forsythus. La lecture rapide des résultats se fait par réaction colorimétrique. Cependant, il est admis que le nombre relativement limité de bactéries concernées par ce test en réduit l'utilité.

Ces tests désignent les sites parodontaux hébergeant des bactéries réputées pathogènes, en donnant des informations à propos de la conduite à tenir en matière de prescription, de suivi d'efficacité de traitement et de mise en place de stratégie de soutien et prévention parodontale. Ces renseignements complémentaires, bien que précieux, restent partiels: le nombre de pathogènes détectés est restreint et ne désigne pas obligatoirement les responsables.

### 3-2-2- Les tests génétiques

Nous avons à maintes reprises souligné (7, 9', 29, 96,...) le rôle des cytokines pro-inflammatoires, du TNF-α, et l'interleukine-1 dans le développement des maladies inflammatoires chroniques, y inclus les parodontites. Ces cytokines présentent un intérêt tout particulier dans le contexte parodontal, du fait de leur interaction avec le métabolisme osseux et leurs propriétés inflammatoires.

IL-1 a été étroitement étudiée à cause de son effet inducteur majeur dans la pathogénie de la maladie parodontale. Des concentrations élevées de IL-1β ont été relevées dans le fluide gingival et les tissus gingivaux chez des patients atteints de parodontites chroniques. Les polymorphonucléaires buccaux produisent une quantité importante de IL-1β. Les niveaux élevés de cytokines contribuent à la susceptibilité à la maladie. Ces variations s'expliquent par une fréquence accrue des génotypes de IL-1β comportant l'allèle 2 du polymorphisme bi-allélique de la longueur des fragments de restriction de IL-1β.

D'autre part, il a été noté une association hautement significative entre l'allèle 2 de l'IL-1β⁺³⁹⁵³ et l'allèle 2 de l'IL -1α⁻⁸⁸⁹, tant chez les malades que les sujets témoins.(29) Les variations de résultats concernant la production de IL-1β semblent liées en grande partie aux méthodes de détection et aux variations inter individuelles de production de cytokines.

La fréquence accrue des génotypes 1/2 et 2/2 de l'IL -1β⁺³⁹⁵³ chez les patients atteints de parodontites sévères comparée à celle observée chez les patients atteints de parodontites modérée ou débutante suggère que l'allèle 2, en corrélation prévisible avec la production accrue de l'IL-1β, pourrait prédisposer à l'accentuation du caractère sévère de la maladie.(29)

Même si le mécanisme, par lequel l'allèle 2 de l'IL-1 β⁺³⁹⁵³ opère, reste mal défini, il n'en reste pas moins qu'il doive agir indépendamment ou interagir de façon ponctuelle avec un ou plusieurs gènes impliqués dans le développement des formes les plus sévères de maladie parodontale, généralement dans les cas où les autres facteurs de risque de déclarer la maladie sont déjà réunis.(29)

Le test génétique s'appuie sur le polymorphisme génétique qui caractérise les variations de réponse de l'hôte à l'activité microbienne. Il permet donc d'évaluer la prédisposition d'un patient à développer une maladie parodontale à tendance sévère et/ou très évolutive, même si les symptômes ne sont pas encore observables.

D' autre part, il trouve toute son utilité dans le dépistage familial de patient à risque déclaré ou pas, dans l'appréciation du degré de vigilance et l'évaluation plus fine du pronostic.

Le test PST+ (Periodontal Susceptibility Test) détecte des variations spécifiques des gènes codant pour l'interleukine II-1α et II-1β.L'intérêt de ce test est multiple:
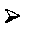 mettre en place précocement une stratégie de prévention dans la mesure où les signes avérés de la maladie ne sont pas encore cliniquement manifestes.
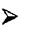 évaluer le pronostic au traitement développé en tenant compte de la capacité biologique du patient à répondre favorablement au protocole.
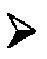 présumer si la maladie parodontale risque d'évoluer selon un mode chronique ou selon un mode progressif voire agressif.
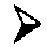 présumer du niveau d'activité de la maladie parodontale ou du risque implantaire.

L'invention concerne donc les procédés suivants, chaque procédé étant pris seul ou en combinaison avec au moins un des autres procédés :
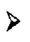 Procédé de détection de la maladie parodontale utilisant des tests bactériens et génétiques dans lequel on détecte des variations spécifiques des gènes codants pour IL-1 α⁻⁸⁸⁹ et IL-1β⁺³⁹⁵³
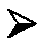 Procédé de détection de la maladie parodontale utilisant des tests bactériens et génétiques dans lequel on mesure l'allèle 2 de l'IL-1α⁻⁸⁸⁹ et/ou l'allèle 2 de l'IL-1β⁺³⁹⁵³
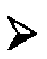 Procédé de détection de la maladie parodontale utilisant des tests bactériens et génétiques dans lequel l'allèle 2 de l'IL-1 β⁺³⁹⁵³ interagit indépendamment ou avec un ou plusieurs gènes impliqués dans le développement des formes les plus sévères de maladie parodontale.
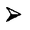 Procédé de détection de la maladie parodontale utilisant des tests bactériens et génétiques dans lequel le test génétique s'appuie sur le polymorphisme génétique qui caractérise les variations de réponse de l'hôte à l'activité microbienne, permettant d'évaluer la prédisposition d'un patient à développer une maladie parodontale à tendance sévère et/ou très évolutive, et ce même si les symptômes ne sont pas encore observables.
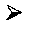 Procédé de détection de la maladie parodontale utilisant des tests bactériens et génétiques dans lequel le test génétique est un test génétique d'évaluation de la prédisposition des sujets testés au développement d'une maladie parodontale à expression inflammatoire.
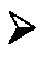 Procédé de détection de la maladie parodontale utilisant des tests bactériens et génétiques dans lequel l'on combine les test de présence de bactéries en milieu buccal et les test génétiques pour évaluer la susceptibilité d'un patient à une maladie parodontale.
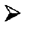 Procédé de détection tel que Id test génétique est le test PST+ (Periodontal Susceptibility Test) qui détecte des variations spécifiques des gènes codant pour l'interleukine II-1α et II-1β.

De préférence, tout procédé selon l'invention est effectué in vitro.

### 3.2.3 Autres tests d'évaluation des défenses de l'hôte

Les produits de dégradation des tissus, les enzymes présents dans le fluide gingival et la salive, sont des indicateurs de la réponse de l'hôte à l'agression microbienne qui engendre une augmentation quantifiable de ces molécules dans les fluides buccaux. La dégradation tissulaire provoque aussi le déversement de substances dans le fluide gingival (débris, médiateurs de l'inflammation tels III-β, TNF-α, PGE2), particulièrement dans les phases d'activité de la maladie.

*L'aspartate amino-transférase* (AST) est un enzyme relargué par les cellules mortes au cours de la maladie parodontale. Les variations des taux enzymatiques dans le fluide gingival laissent à penser que ce dosage pourrait être un indicateur de la maladie parodontale. Cependant, les taux restent élevés pour une gingivite ou une parodontite de l'adulte, même stabilisée. Ce moyen d'évaluation perd donc son intérêt.

*Les protéases neutres* sont des enzymes lysosomiaux déversés en grande quantité par les polymorphonucléaires neutrophiles (PMN) à leur contact avec les bactéries. L'élastase du fluide a été dosée au cours de la maladie parodontale, avec une sensibilité de 84% et une spécificité de 66% pour l'aggravation de la maladie. Cette utilisation présente un intérêt portant sur le dépistage précoce d'une activité inflammatoire des PMN, proportionnelle à l'évolution de la maladie.

### 3.3 Une autre lecture: définition biologique des maladies parodontales

Quels sont ces caractères étiologiques?

Le développement présent a aussi eu pour objet de faire un point, le plus exhaustif possible, sur les causes aussi variées fussent-elles, des malades parodontales d'étiologie bactérienne. Il semble, à ce jour, que les domaines soient à peu près circonscrits, même si persistent encore de nombreuses interrogations, pour clarifier les éléments composant chacun d'eux.

La difficulté majeure reste les interrelations entre les différents paramètres intervenant dans le diagnostic étiologique. Faire un point serré, demande une grande précision d'analyse et un questionnaire médical très fouillé.

Nous relevons donc les paramètres suivants:
- La flore microbienne, nature et localisation
- Les composants salivaires
- La chaîne réactionnelle inflammatoire et ses variations
- La réponse immunitaire et ses variations
- Le caractère génétique et ses variations
- Les facteurs exogènes: stress, tabagisme, alimentation, mode de vie
- Le stress oxydatif
- Les syndromes associés à une expression de la maladie parodontale

### 4 Définition d'un indice d'activité sur la base de:

***M***odèle ***A***ctualisé de ***R***echerche de ***C***ritères pour un ***I***ndice d'***A***ctivation

### Désigna tion

**Indice: Idam**

**Index: madl**

***M***odèle ***A***ctualisé de ***R***echerche de ***C***ritères pour un ***I***ndice d' ***A***ctivation (ou Indice diagnostic d'activation marcia: Idam)

(updated criterions research model for activation index: marcia activation diagnosis Index: madl )

| INDICE D'ACTIVATION / *ACTIVATION INDEX* |
|---|
| DEFINITION |
| Étalonnage de la réactivité présumée de " l'unité diagnostique parodontale " telle qu'elle a été définie, prenant en compte la pathogénicité / virulence de la flore microbienne, les caractéristiques génétiques et environnementales du patient. L'unité diagnostique parodontale étant composée du " complexe parodontal unitaire moyen " pour un patient donné, à un instant donné, relativement aux critères pris en compte. Le " complexe parodontal unitaire moyen " étant constitué des tissus cémentaires, ligamentaire et ostéo-muqueux autour d'une dent théorique cumulant les caractéristiques estimées pour le patient considéré, au moment de l'examen parodontal. Pour des raisons de commodité, nous choisirons une valeur numérique de l'indice facile à utiliser et suffisamment implicite pour exprimer le degré de morbidité parodontale. Au préalable, les différents critères retenus seront affublés d'une valeur subjective empirique mais reproductible de façon à composer une sorte d'algorithme simpliste traduisant l'impact de chaque critère sur la définition globale de l'entité " patient - maladie - traitement - pronostic ". Pour des raisons épidémiologiques flagrantes, cet indice peut être affiné en respectant le principe général de sa définition. Nous parlerons de périodontite d'indice de **I** à VIII ou *parodontitis from I to VIII index.* |

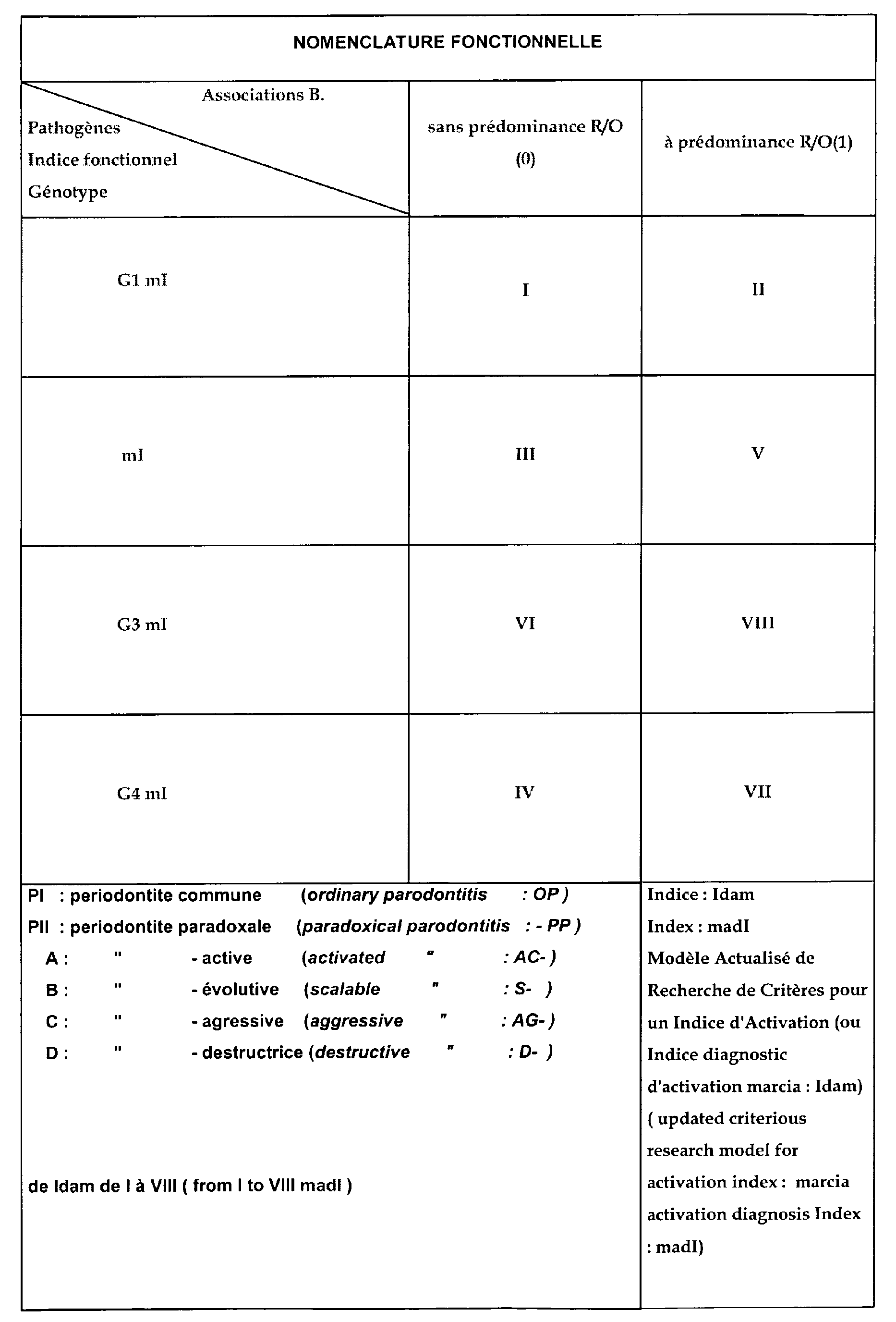

### 3-5- Principes d'une nomenclature fonctionnelle des maladies parodontales. Classification fonctionnelle des expressions biocliniques parodontales

Quatre caractères fonctionnels émergent constamment dans la description des maladies parodontales d'étiologie microbienne.
- Activité
- Evolutivité
- Agressivité
- Destruction

La question clé qui se pose est la suivante:
Au-delà de l'étiologie locale bactérienne incontestée de la maladie parodontale, comment rendre compte le plus rationnellement possible, des autres facteurs causaux de la maladie?

Un organisme génétiquement apte à exprimer une immunité efficace est-il capable de juguler le développement d'une flore pathogène?

La prolifération d'une flore, quelle que soit sa pathogénicité, n'est que la conséquence écologique d'une réactivité plus ou moins favorable de l'écosystème dans lequel elle évolue, croît et diffuse ses substrats cataboliques. Toute population biologique s'étend dans la stricte limitation de ses critères de développement.
Quels sont ces critères de développement?
Est- il possible aujourd'hui d'établir un descriptif satisfaisant, donc épidémiologique, de ces critères?

La lecture des communications les plus récentes nous apparaît comme prometteuse, parce que innovante. Chacune, dans un registre de spécialité tout à fait ciblé, nous apporte des éléments de réflexion qui, associés, nous incitent à élaborer un nouveau schème.

Tenant compte de tous ces caractères, l'expression pathologique prend une toute autre signification, sans doute bien au- delà de la lecture des indices habituellement référencés en parodontie.

Notre compréhension de la pathognomonie et de la nature microbiologique de la maladie parodontale, risque d'être désormais limitée, si nous nous cantonnons à un examen et une analyse des paramètres cliniques ou fonctionnels trop peu explicites, parce que strictement symptomatiques.
Qu'est ce qui nous a conduits à évoquer des manifestations «réfractaires» à tout traitement?
Les données de l'époque sur lesquelles était fondée cette appréciation, n'apparaissent-elles pas aujourd'hui trop partielles?

Actuellement, l'approche épidémiologique a tendance à se référer à des facteurs essentiellement périphériques, suspectés d'être prédisposant à la maladie parodontale, sans pour autant être, par définition, obligatoirement associés à une dysfonction parodontale avérée.
Ces caractères, potentiellement, présentent une capacité d'altérer considérablement l'expression de l'arbre fonctionnel du processus inflammatoire.
Au point qu'une flore commensale peut apparaître "pathogène"; non pas que ses critères propres en soient modifiés, mais que la défaillance, constitutive ou acquise de la réponse de l'hôte, accentue vraisemblablement sa virulence apparente.

Certes, toutes les manifestations ne sont pas du même registre de gravité.

Certes, les désordres observés dans les formes agressives voire destructrices ne représentent qu'un faible pourcentage de l'expression de la maladie.

Cependant, il est flagrant que ces cas à risque nous préoccupent singulièrement et représentent la cible de notre plus forte vigilance y compris en stratégie de soutien.

Quand bien même l'approche «classique» de la thérapeutique parodontale concernerait la plupart des patients en besoin de soins, pouvons nous, devons nous traiter, selon les mêmes protocoles, ceux à l'immunité ou à la génétique intrinsèquement ou transitoirement défaillantes?

En aucune façon, le test génétique a pour ambition de contribuer au diagnostic d'une forme avérée de la maladie parodontale, mais désigne un contexte biologique, expression directe d'une identité génétique du patient.

L'expression du gène biloculaire codant la production d'interleukine, selon son degré amplifié ou pas, et/ou la mutation du gène récepteur, désigne ou pas les sujets prédisposés à développer une maladie parodontale. Sa sévérité sera, bien entendu, fonction en premier lieu de la flore pathogène recensée, tous critères largement évoqués confondus et des facteurs locaux, régionaux, généraux ou environnementaux modifiant l'écosystème bucco-dentaire au sens très large.

Une revue de la littérature nous a conduits à élaborer le tableau suivant, faisant état de critères majeurs systématiquement rencontrés à des degrés divers, qui caractérisent la manifestation de la maladie parodontale. La conjonction de ces paramètres aboutit à, soit une expression clinique de la maladie, soit à l'énoncé d'une susceptibilité à la maladie.

Dans ce cas, est présumé le degré possible d'activité, d'agressivité, voire de destruction tissulaire.

**Définition actuelle de la maladie parodontale et les bases des différentes approches diagnostiques et thérapeutiques.** (2 ,3) Ou **De l'impact des nouvelles données acquises par leur application en thérapeutique parodontale.**Enumération épidémiologique des différents facteurs déclenchant ou favorisant la maladie parodontale.(4,7,13,25,34,37,47,53,66,67,84,86,107,111,117).

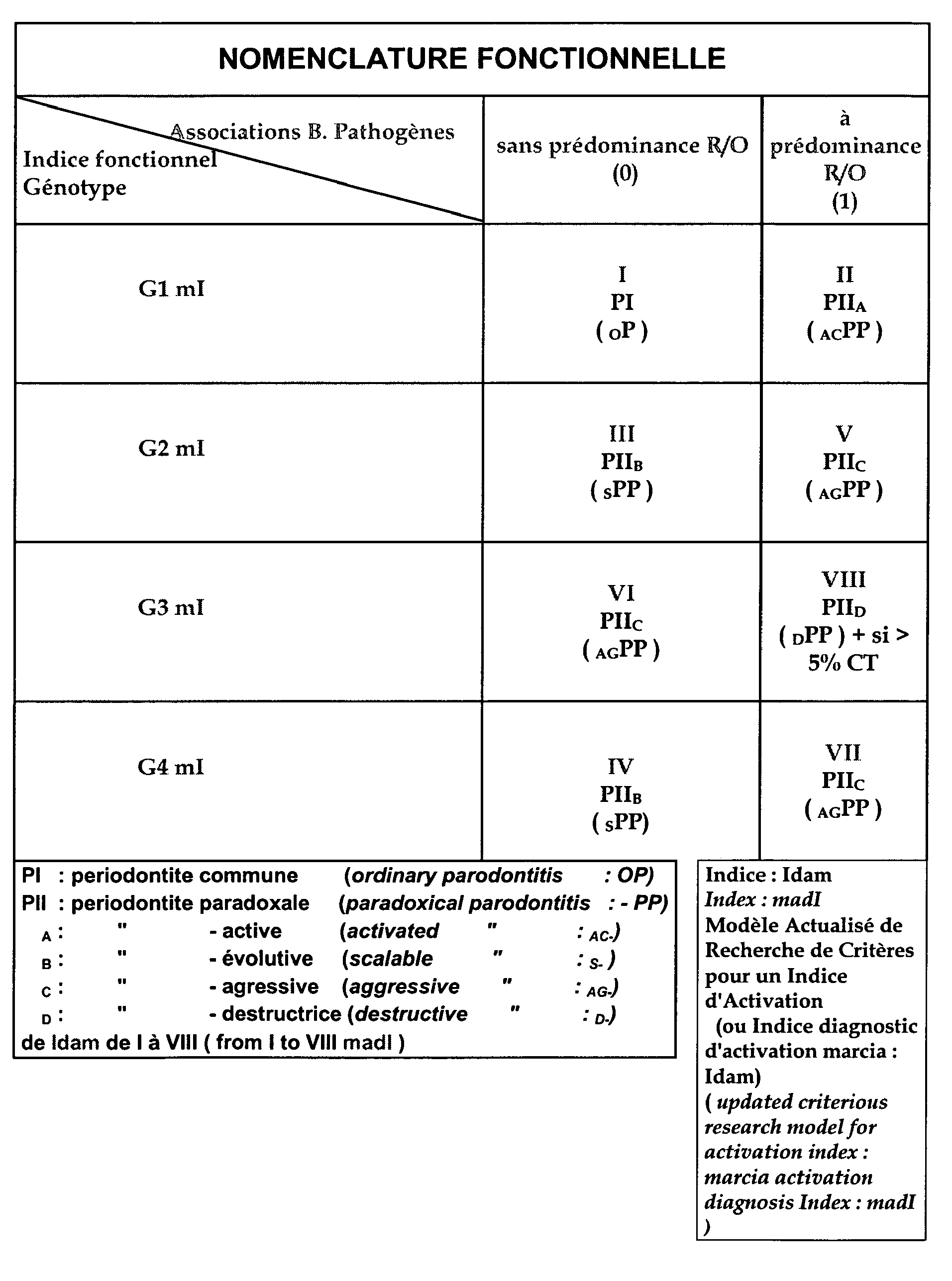

Nomenclature fonctionnelle des maladies parodontales d'origine microbienne:

| | |
|---|---|
| **Périodontite commune (PI)** | **(mI I)** |
| **Périodontite Paradoxale Active (PII_{A})** | **(mI II)** |
| **Périodontite Paradoxale Evolutive (PII_{B})** | **(mI III)** |
| **Périodontite Paradoxale Evolutive (PII_{B})** | **(mI IV)** |
| **Périodontite Paradoxale Agressive (PII_{C})** | **(mI V)** |
| **Périodontite Paradoxale Agressive (PII_{C})** | **(mI VI)** |
| **Périodontite Paradoxale Agressive (PII_{C})** | **(mI VII)** |
| **Périodontite Paradoxale Destructrice (PII_{D})** | **(mI VIII)** |

| | |
|---|---|
| **Ordinary Parodontitis (OP)** | **(mI I)** |
| **Activated Paradoxical Parodontitis (_{AC}PP)** | **(mI II)** |
| **Scalable Paradoxical Parodontitis (_{S}PP)** | **(mI III)** |
| **Scalable Paradoxical Parodontitis (_{S}PP)** | **(mI IV)** |
| **Aggressive Paradoxical Parodontitis (_{AG}PP)** | **(mI V)** |
| **Aggressive Paradoxical Parodontitis (_{AG}PP)** | **(mI VI)** |
| **Aggressive Paradoxical Parodontitis (_{AG}PP)** | **(mI VII)** |
| **Destructive Paradoxical Parodontitis (_{D}PP)** | **(mI VIII)** |

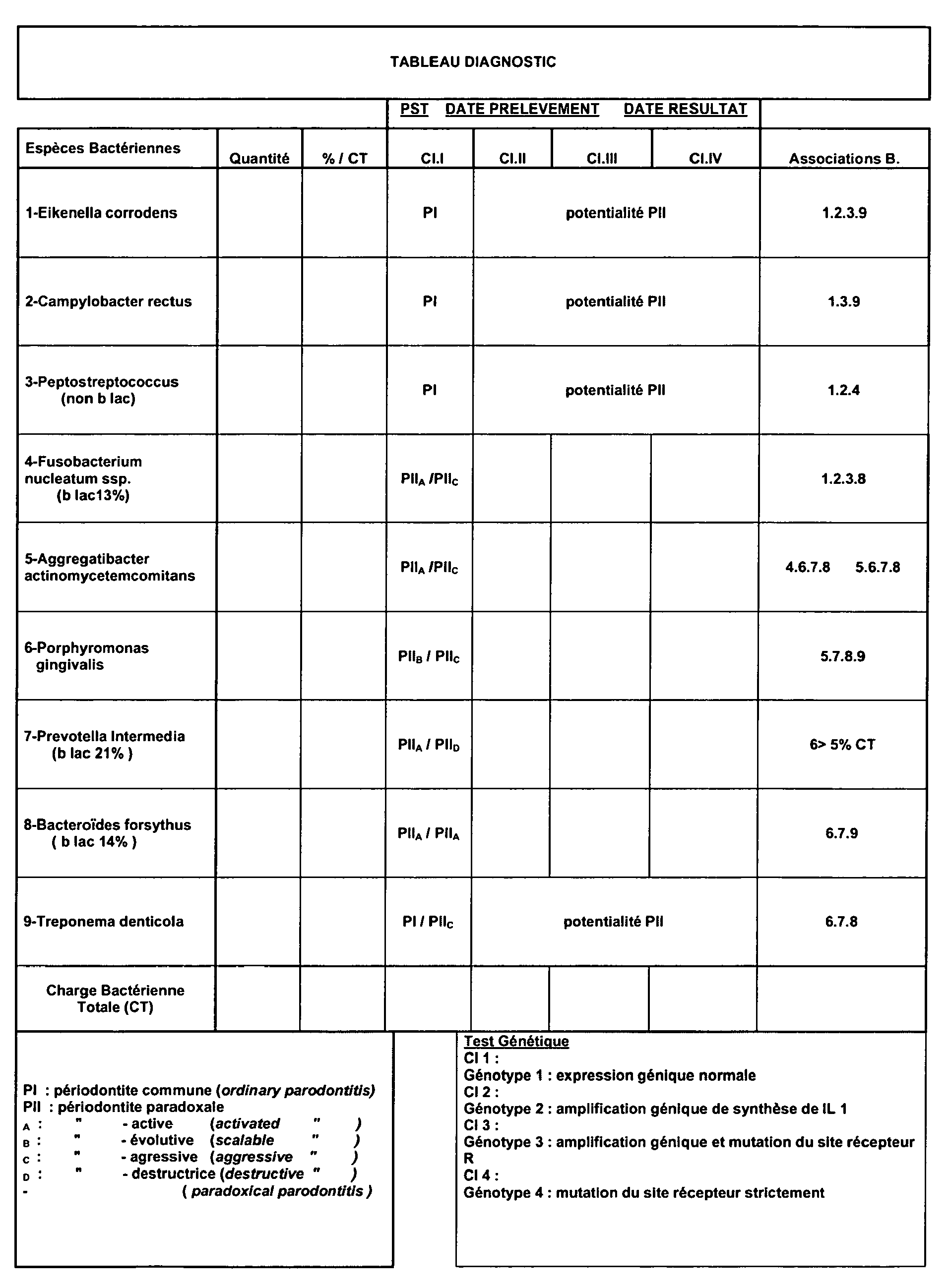

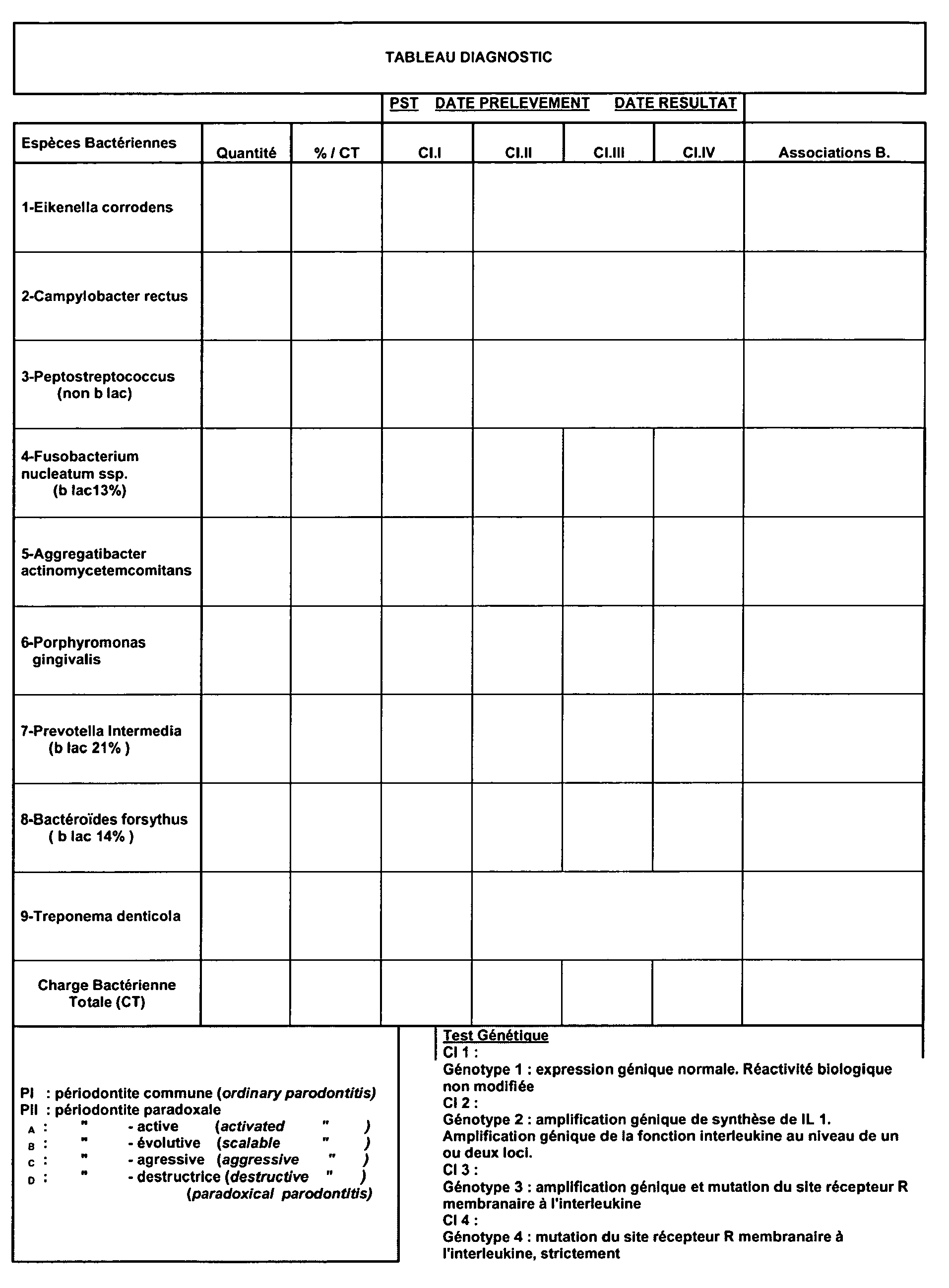

### BIBLIOGRAPHIE

1- ABRAMSON J.L., HOOPER W. C., JONES D. P., ASHFAQ S., RHODES S.D., WEINTRAUB W.S., HARRISON D.G., QUYYAMI A., VACCARINO V. Association between novel oxidative stress markers and C- reactive protein among adults without clinical coronary heart disease. Atherosclerosis 178 (2005) 115-121.
2- ARMITAGE G. C. Clinical evaluation of periodontal diseases. Periodontol. 2000, Vol.7, 1995, 39-53.
3- ARMITAGE G C. Development of a classification system for periodontal diseases and conditions. Ann Periodontol 1999;4:1-6.
4- BARBIERI B. Premières adhesions bacteriennes dans le biofilm dentaire. JPIO Vol. 26.3; 201-211.
5- BECK J. D. Issues in assessment of diagnostic tests and risks for periodontal diseases. Periodontol. 2000, Vol. 7, 1995, 100-108.
6- BERGLUNDH T., DONATI M., ZITZMANN N. Bcells in periodontitis- friends or ennemies? Periodontol 2000, Vol. 45, 2007, 51-66.
7- BLOQUEL C. Thérapie génique non virale de variants du gène du récepteur soluble de type I du TNF- alpha humain. These pour l'obtention du diplôme de Docteur de l'Université Paris 7 Spécialité: Biologie de l' os et des articulations et biomatériaux des tissus calcifiés. 07- 06- 2005
8- BODET C., CHANDAD F., GRENIER D. Pathogenic potential of Porphyromonas gingivalis, Treponema denticola and Tannerella forsythia, the red bacterial complex associated with periodontitis. Pathologie Biologie, Vol. 55, issues 3-4,april- may 2007; pp 154- 162.
9- BONFILL JJ, DILLIER FL, MERCIER P et al. A "case control" study on the role of HLA- DRA in severe periodontitis and rapidly progressive periodontitis. Identification of types and subtypes using molecular biology (PCR ASSO).J Clin Periodontol 1999;26:77-84.
9'- CAFFESSE R.G., DE LA ROSA R., DE LA ROSA G.M., WELTMAN R. Effect of interleukin-1 gene polymorphism in a periodontally healthy Hispanic population treated with mucogingival surgery. J. Clin. Periodontol 29 (2), 177-181.
10-CHALLACOMBE Sj, WILTON JMA.A study of antibodies and opsonic activity in human crevicular fluid in relation to periodontal disease. J Periodontol Res 1984: 19: 604- 608.
11-CHAPPLE I. L. C., MATTHEWS J. B. The role of reactive oxygen and antioxydant species in periodontal tissue destruction. Periodontol. 2000, Vol. 43, 2007, 160- 232.
12-COMBARROS O., INFANTE J., LLORCA J., BERCIANO J. Interleukin- 1A(-889) Genetic Polymorphism Increases the risk of multiple system Atrophy. Movement Disorders, Vol.18, N° 11, 2003.(cnrs)
13-CROUCHER R., MARCENES W. S., TORRES M. C.M. B., HUGHES W. S., SHEIHAM A. The relationship between life- events and periodontitis. J Clin Periodontol 1997 ; 24: 39-43.
14- CURTIS M. A., SLANEY JM., ADUSE-OPOKU J. Critical pathways in microbial virulence. J. Clin. Periodontol. 2005;32(supp 6) : 28-38.
15- CUTLER C. W., TENG Y-T. A. Oral mucosal dendritic cells and periodontitis: many sides of the same coin with new twists. Peridontol.2000, Vol. 45, 2007, 35- 50.
16- DARVEAU R. P., TANNER A., PAGE R. C. The microbial challenge in periodontitis. Periodontol. 2000, Vol. 14, 1997, 12- 32.
17- DAVEY M. E. Techniques for the growth of Porphyromonas gingivalis biofilms. Periodontol. 2000, Vol. 42, 2006, 27-35.
18- DAVEY M. E., COSTERTON J. W. Molecular genetics analyses of biofilm formation in oral isolates. Periodontol. 2000, 2006, Vol. 42, 13- 26.
19-DENNISON D. K., VAN DYKE T. E. The acute inflammatory response and the role of phagocytic cells in periodontal health and disease. Periodontol. 2000, Vol. 14, 1997, 54-78.
20- DUBREUIL L., MOUTON Y. Antibiothérapie en odonto- stomatologie Diaporama Service Regional Universitaire des Maladies Infectieuses et du Voyageur Centre hospitalier de Tourcoing. 7-12-2002.
21- EBERSOLE J.L., TAUBMAN M.A. The protective nature of host responses in periodontal diseases. Periodontol. 2000, Vol. 5, 1994; 112-141.
22- FINE D. H., KAPLAN J. B., KACHLANY S. C., SCHREINER H. C. How we got attached to Actinobacillus actinomycetemcomitans: a model for infectious diseases. Periodontol. 2000, Vol. 42, 2006, 114- 157.
23-FLETCHER RH, FLETCHER SW, WAGNER EH. Clinical epidemiology: the essentials. 2nd edn. Baltimore: WILLIAMS & WILKINS, 1988.
24-GEMMEL E., MARSHALL R.I., SEYMOUR G. J. Cytokines and prostaglandins in immune homeostasis and tissue destruction in periodontal disease. Periodontol. 2000, Vol. 14, 1997, 112- 143.
25-GEMMEL E., YAMASAKI K.., SEYMOUR G. The role of Tcells in periodontal disease: homeostasis and autoimmunity. Periodontol. 2000, Vol. 43, 2007, 14- 40.
26- GJERMO PE. Impact of periodontal preventive programmes on the data from epidemiologic studies. J. Clin. Periodontol. 2005; 32 (Suppl.6):294-300.
27- GILLETT I. R., JOHNSON N. W., CURTIS M. A., GRIFFITH G. S., STERNE J. A. C., CARMAN R. J., BAMPTONJ. L. M., WILTON J. M. A. The role of histopathology in the diagnosis and prognosis of periodontal diseases. J Clin Periodontol 1990; 17: 673-684.
28- GOODSON J. M. Clinical mesurements of periodontitis. J Clin Periodontol. 1986, 13;446-455
29- GORE EA., SANDERS JJ., PANDEY JP., PALESH Y., GALBRAITH GMP. Interleukin-1B+3953 allèle 2: Association with disease status in adult periodontitis. J Clin Periodontol 1998. 25:781-785. (cnrs)
30- GRAMIGNANO G., LUSSO M.R., MADEDDU C., MASSA E., SERPE R., DEIANA L., LAMONICA G., DESSI M., SPIGA C., ASTARA G., MACCIO A., MANTOVANI G. Efficacy of L- carnitine administration On fatigue, nutritional status, oxidative stress, and related quality of life in 12 advanced cancer patients undergoing anticancer therapy. Nutrition 22 (2006) 136- 145.
31-HAN X., KAWAI T., TAUBMAN M. A. Interference with immune- cell- mediated bone resorption in periodontal disease. Periodontol. 2000, Vol. 45, 2007, 76- 94.
32- HAFFAJEE A. D., TELES R. P., SOCRANSKY S. S. The effect of periodontal therapy on the composition of the subgingival microbiota.Periodontol. 2000, Vol. 42, 2006, 219- 258.
34- HAFFAJEE A.D., SOCRANSKY S.S. Microbial etiological agents of destructive periodontal diseases. Periodontol. 2000, Vol. 5, 1994; 78-111.
35- HANIOKA T., TAKAYA K., MATSUMORI Y., MATSUSE R., SHIZUKUISHI S. Relationship of the substance to indicators of host response in human gingival crevicular fluid. J Clin Periodontol, 2000; 27:262-266.
36- HANNING M, HANNING C. Existe- t- il un biofilm dentaire, dépourvu de bacteries, in situ. JPIO. Vol. 26.3; 187- 200.
37- HART T. C., KORNMAN K. S. Genetic factors in the pathogenesis of periodontitis. Periodontol. 2000, Vol. 14, 1997, 202-215.
38- HART T. C., ATKINSON J. C. Mendelian forms of periodontitis. Periodontol. 2000, Vol. 45, 2007, 95-112.
39- HEITZ-MAYFIELD LJA. Disease progression: identification of high-risk groups and individuals for periodontitis J. Clin. Periodontol. 2005; 32 (Suppl. 6) 196-209.
40- HOLDMAN LV, MOORE WEC, CATO EP, BURMEISTER JA, PALCANIS KG, RANEY RR. Distribution of Capnocytophaga in periodontal microfloras. J Periodont Res 1985;20:475-483.
41- HOURI-HADDAD Y., WILENSKY A., SHAPIRA L. T cell phenotype as a risk factor for periodontal disease. Periodontol 2000, Vol. 45, 2007, 67-75.
42- ISHIKAWA I., NAKASHIMA K., KOSEKI T., NAGASAWA T., WATANABE H., ARAKAWA S., NITTA H., NISHIHARA T. Induction of the immune response to periodontopathic bacteria and its role in the pathogenesis of periodontitis. Periodontol. 2000, Vol. 14, 1997, 79- 111.
43- KEITH L, KIRKWOOD K. L., CIRELLI J. A., ROGERS J. E., GIANNOBILE W. V. Novel host response therapeutic approaches to treat periodontal diseases. Periodontol. 2000, Vol. 43, 2007, 294- 315.
44- KILIAN M., FRANDSEN E. V.G., HAUBEK D., POULSEN K. The etiology of periodontal disease revisited by population genetic analysis. Periodontol. 2000, Vol. 42, 2006, 158- 179.
45- KINANE DF, ATTSTRÖM R. Advances in the pathogenesis of periodontitis. Group B consensus report Of the fifth European workshop in periodontology. J. Clin. Periodontol. 2005; 32 (Suppl. 6): 130- 131.
46- KINANE D. F., BARTOLD P. M. Clinical relevance of the host responses of periodontitis. Periodontol. 2000, Vol. 43, 2007, 278- 293
47- KINANE D. F., DEMUTH D. R.? GORR S-V., HAJISHENGALLIS G. N., MARTIN M. H. Human variability in innate immunity. Periodontol.2000,Vol.45,2007,14-34.
48- KINANE D.F., OONEY J., EBERSOLE J.L. Humoral oral immune response to Actinobacillus actinomycetecomitans and Porphyromonas gingivalis in periodontal disease. Periodontol.2000, Vol. 20, 1999; 289-340.
49- KINGMAN A., ALBANDAR J.M. Methodological aspects of epidemiological studies of periodontal diseases. Periodontol.2000, Vol. 29, 2002; 11-30.
50- KLINGE B., NORLUND A. A socio- economic perspective on periodontal diseases: a systemic review. J. Clin. Periodontol. 2005 (Suppl. 6); 314-325.
51- KOLENBRANDER P. E., PALMER Jr R. J., RICKARD A.H., JAKUBOVICS N. S., CHALMERS N., DIAZ P.I. Bacterial interactions and successions during plaque development. Periodontol. 2000, Vol. 42, 2006, 47- 79.
52- KORNMAN K. S., LÖE H. The role of local factors in the aetiology of periodontal diseases. Periodontology 2000 1993,Vol. 2:83-97.
53- KORNMAN K. S., PAGE R. C., TONETTI M., S. The host response to the microbial challenge in periodontitis: assembling the players. Periodontol. 2000, Vol. 14, 1997, 33- 53.
54- LAKHSSASSI N, ELHAJOUI N, LODTER JP, PINEIL JL, SIXOU M. Antimicrobial susceptibility variation of 50 anaerobic periopathogens in aggressive periodontitis: an interindividual variability study. Oral Microbiol Immunol 2005a; 20:244-252.
55- LAKHSSASSI N, SIXOU M. Variabilité de l'efficacité de l'erythromycine et de la spiramycine sur les pathogènes parodontaux dans les parodontites agressives. Etude in vitro comparative. Pathol Biol 2005b; 53: 527-537.
56- LAMSTER I. B. Diagnosis of periodontal disease based on analysis of the host response. Periodontol. 2000, Vol. 7, 1995, 83-99.
57- LANG NP., LINDHE J., VAN DER VELDEN U. on behalf of the European Workshop in Periodontology group D. J. Clin. Periodontol. 2005; 32 (Suppl. 6); 2916293.
58- LOOS BG., JOHN RP., LAINE ML. Identification of genetic risk factors for periodontitis and possible mechanisms of action. J. Clin. Periodontol. 32 (Suppl. 6); 159-179.
59- MADIANOS PN., BOBETSIS YA., KINANE DF. Generation of inflammatory stimuli: how bacteria set up inflammatory responses in the gingival. J. Clin. Periodontol. 2005; 32 (Suppl. 6); 57-71.
60- MAHANONDA R., PICHYANGKUL S.. Toll- like receptors ant their role in periodontal health and disease. Periodontol. 2000, Vol. 43, 2007, 41- 55.
61- MANTOVANI G., MADEDDU C., GRAMIGNANO G., LUSSO M.R., MOCCI M., MASSA E., FERRELI L., ASTARA G ., MACCIO A., SERPE R. Subcutaneous Interleukin- 2 in combination with medroxyprogesterone acetate and antioxidants in advanced cancer responders to previous chemotherapy: phase II study evaluating clinical, quality of life, and laboratory parameters. J. of Exp. Therap. and Oncol. Vol. 3:205-219, 2003.
62- MANTOVANI G., MADEDDU C., MACCIO A., GRAMIGNANO G., LUSSO M.R., MASSA E., ASTARA G., SERPE R. Cancer-Related Anorexia/ Cachexia Syndrome and Oxidative Stress : An Innovative Approach beyond Current Treatment. Cancer Epidemiol Biomarkers Prev 2004;13(10). 10. 2004.
63- MARSH PD. Dental plaque: biological significance of a biofilm and community life- style. J. Clin. Periodontol. 2005; 32 (Suppl. 6); 7-15.
64- MASADA MP, PERSSON R, KENNEY JL, LEE SW, PAGE RC, ALLISON AC. Measurement of interleukin-1 alpha and - 1 beta in gingival crevicular fluid: implications for the pathogenesis of periodontal disease. J Periodontol Res 1990: 25: 156-163.
65- MATSUKI Y, YAMAMOTO T, HARA K. Interleukin-1 mRNA- expression macrophages in human chronically inflamed gingival tissues. Am J Pathol 1991: 138: 1299- 1305.
66- MENG H., LI Q., HAN J., ZHAO Y. Determinants of host susceptibility in aggressive periodontitis. Periodontol. 2000, Vol. 43; 2007, 133- 159.
67- MEURETTE O. Apoptose et mort cellulaire: Mécanismes généraux et regulation. INSERM U620. Internet
68- MOORE W.E.C., MOORE L.V.H. The bacteria of periodontal diseases. Periodontol. 2000, Vol. 5,1994, 66-77.
69- MURAYAMA Y., KURIHARA H., NAGAI A., DOMPKOWSKI D., VAN DYKE E. T. Acute necrotizing ulcerative gingivitis: risk factors involving host defense mechanisms. Periodontol. 2000 , Vol 6, 1994, 116-124.
70- NAGASAWA T., KIJI M.,YASHIRO R., HORMDEE D., HE L., KUNZE M., SUDA T., KOSHY G., KOBAYASHI H., ODA S., NITTA N.H., SHIKAWA I. Roles of receptor activator of nuclear factor- kB ligand (RANKL) and osteoprotegerin in periodontal health and disease. Periodontol. 2000, Vol. 43, 2007, 65- 84.
71- NEEDLEMAN I., SUVAN J., MOLES DR. , PIMLOTT J. Asystematic review of professional mechanical plaque removal for prevention of periodontal diseases. J. Clin. Periodontol. 2005 ; (Suppl. 6) ;229-282.
72- NETUSCHIL L, HOFFMANN T, BRECX M. Bactéries de la salive, bactéries des biofilms dentaires. JPIO, Vol. 26, 3;08-2007. 177- 186.
73- NOGUSHI K., ISHIKAWA I. The role of cyclooxygenase- 2 and prostaglandin E2 in periodontal disease. Periodontol. 2000, 43, 2007, 85-101
74- OFFENBACHER S, ODLE B. M., VAN DYKE T. E. The use of crevicular fluid prostaglandin E2 levels as a predictor of periodontal attachment loss. J Periodont Res 1986: 21: 101-112.
75- PAGE R. C., KORNMAN K. S. The pathogenesis of human periodontitis: an introduction. Periodontol. 2000, Vol. 14, 1997, 9-11.
76- PAGE R. C., OFFENBACHER S., SCHROEDER H;E., SEYMOUR G. J., KORNAM S. Advances in the pathogenesis of periodontitis: summary of developments, clinical implications and future directions. Periodontol. 2000, VOL. 14, 1997, 216- 248.
77- PALMER RM., WILSON RF., HASAN AS., SCOTT DA. Mechanisms of action of environmental factors -tobacco smoking. J. Clin. Periodontol. 2005;(Suppl. 6);180-195.
78- PASTER B. J., OLSEN I., AAS J.A., DEWHIRST F.E. The breadth of bacterial diversity in the human periodontal pocket and other oral sites. Periodontol 2000, Vol. 42, 2006, 80-86.
79- PERSSON RG. Immune responses and vaccination against periodontal infections. J. Clin. Periodontol. 2005 32 (Suppl.6);39-53.
80- RAMSEIER CA. Potential impact of subject-based risk factor control on periodontitis. J. Clin. Periodontol. 2005.32 (Suppl. 6); 283-290.
81- REYNOLDS J. J., MEIKLE C. Mechanisms of connective tissue matrix destruction in periodontitis. Periodontol. 2000, Vol. 14, 1997, 144-157.
82- ROBERTS A. P., MULANY P. Genetic basis of horizontal gene transfer among oral bacteria. Periodontol. 2000, Vol. 42, 2006, 36-46.
83- RYDER M. I., The influence of smoking on host responses in periodontal infections. Periodontol. 2000, Vol. 43, 2007, 267-277.
84- SAIDI- OUAHRANI N., BOUZIANE D. Hérédité des parodontites agressives. JPIO Vol. 26 N°2; 05.2007;137-139
85- SALVI G., LENG N.P. Therapeutic modulation of the host response in the management of periodontal diseases. J. Clin. Periodontol. 32 (Suppl. 6);2005.
86- SALVI G. E., LAWRENCE H. P., OFFENBACHER S., BECK J. D. Influence of risk factors on the pathogenesis of periodontitis. Periodontol. 2000, Vol. 14, 1997, 173-201.
87- SANZ M., QUIRINEN M. on behalf of the European Workshop in Periodontology group A J. Clin. Periodontol. 2005; (Suppl. 6); 54-56.
87'- SFPIO Region Val de Loire. MICHEL J.F.; HOURDIN S. Actualisation du plan de traitement.26-01-2008 Nantes.
88- SHAPIRA L., WILENSKY A., KINANE DF. Effect of genetic variability on the inflammatory response to Periodontal infection. J. Clin. Periodontol. 2005;32(Suppl. 6);72-86.
89- SCHENKEIN H. A., BARBOUR S.E., TEW J. G. Cytokines and inflammatory factors regulating immunoglobulin production in aggressive periodontitis. Periodontol. 2000, Vol. 45, 2007,113-127.
90- SCHULLER A.A., HOLST D. Testing the consistency of measurements of rhe distance between the cemento- enamel junction and the alveolar bone crest on bitewing radiographs. J Clin Periodontol 1996; 23: 977-981.
91- SIXOU M, DIOUF A, LAKHSASSI N. Différentes flores bactériennes pour différentes entités de la maladie parodontale. JPIO Vol. 26.3; 233- 244.
92- SLOTS J. Bacterial specificity in adult periodontitis. J Clin Periodontol 1986b;13:912-917.
93- SOCRANSKY SS, HAFFAJEE AD. Periodontal microbial ecology. Periodontolgy 2000 2005; 38: 135- 187.
93'- SOCRANSKY SS, HAFFAJEE AD, CUGINI MA, SMITH C, KENT Jr RL. Microbial complexes in subgingival plaque. J Clin Periodontol 1998; 25: 134- 144.
94- SOCRANSKY SS, TANNER ACR, HAFFAJEE AD, HILLMAN JD, GOODSON JM. Present status of studies on the microbial etiology of periodontal diseases. in : GENCO RJ, MERGENHAGEN S.E., eds. Host- parasite interactions in periodontal diseases. WASHINGTON DC: American Society for Microbiology, 1980:1- 12.
95- SMITH QT, HINRICHS JE, MELNYK RS. Gingival crevicular fliud myeloperoxidase at periodontitis sites. J Periodont Res 1986: 21:45-55.
96- TANNER A.C., IZARD J. Tannerella forsythia, a periodontal pathogen entering the genomic era. Periodontol. 2000, Vol. 42, 2006, 88-113.
97- TANNER A., MAIDEN M.F.J., PASTER B.J., DEWHIRST F.E. The impact of 16S ribosomal RNA-based phylogeny on the taxonomy of oral bacteria. Periodontol. 2000, Vol. 5, 1994; 26-51.
98- TONNETTI MS., CLAFFEY N. Advances in the progression of periodontitis and proposal of definitions of a periodontitis case and disease progression for use in risk factor research. Group C Consensus report of the fifth European Workshop in Periodontology. J. Clin. Periodontol. 2005 ; 32(Suppl. 6); 210-213.
99- TEDGUI A., MALLAT Z. Inflammation et athérosclérose. Actualités néphrologiques. FLAMMARION- MEDECINE-SCIENCES.
100- TELES R. P., HAFFAJEE A.D., SOCRANSKY S. S. Microbiological goals of periodontal therapy. Periodontol. 2000, Vol. 42,2006, 180- 218.
102- TENG Y. T. A., TAYLOR G. W. , SCANNAPIECO F., KINANE D. F., CURTIS M., BECK J. M., KOGON S. Santé parodontale et troubles systémiques. J Can Dent Assoc 2002; 68(3): 188-192. Cet article a fait l'objet d'une révision par des pairs.
103- UDAGAWA N., SATO N.., YANG S., NAKAMURAM., YAMASHITA T., NAKAMURA H., NOGUSHI T.. Signal transduction of liposaccharide-induced osteoclast differenciation. Periodontol. 2000, Vol. 43, 2007, 56- 64.
104- Université de RENNES 1 U.F.R., Faculté d' Odontologie. Maladies générales et parodontopathies.
   Question d' Internat N° 97. Mise à jour 23.03.2004
105- Université de RENNES 1 U.F.R., Faculté d'Odontologie. Epidémiologie des maladies parodontales.
   Question d'Internat N° 128. Mise à jour 04.03.2003
106- VAN DER WEIJDEN GA., HIOE KPK. A systemic review of the effectiveness of self-performed mechanical plaque removal in adults with gingivitis using a manual toothbrush. J. Clin. Periodontol. 2005;(Suppl. 6); 214-228.
107- VAN DYKE T.E. Cellular and molecular susceptibility determinants for periodontitis. Periodontol 2000, Vol.45, 2007, 10-13.
108- VAN DYKE T. E. Control of inflammation and periodontitis. Periodontol. 2000, Vol. 45, 2007, 158- 166.
   108'- VON OHLE C, BRECX M. Formation initiale du biofilm dentaire chez I' homme. JPIO Vol. 26; 3;213- 231.
109- VAN WINKELHOFF AJ., BOUTAGA K. Transmission of periodontal bacteria and models of infection. J. Clin. Periodontol. 32;(Suppl. 6); 16-27.
110- VON OHLE C., BRECX M. Early human dental biofilm formation. JPIO Vol. 26. N°3; 08;2007.
111- YAMADA M., IKEGAMI A., KURAMITSU H. Synergistic biofilm formation by Treponema denticola And Porphyromonas gingivalis. FEMS Microbiology letters, 2005, Vol. 250, n°2;pp 271-277. (cnrs)
112- YOSHIE H.., KOBAYASHI T.., TAI H., GALICIA J. C. The role of genetic polymorphisms in periodontitis. Periodontol. 2000, Vol. 43, 2007, 102- 132.
113- ZAPPA U. Histology of the periodontal lesion: implications for diagnosis. Periodontol. 2000, Vol. 7, 1995, 22-38.
114- ZAPPA U, REINKING- ZAPPA M, GRAF H, ESPELAND M. Cell populations and episodic periodontal attachment loss in humans. J Clin.Periodontol 1991: 18: 508- 515.
115- ZAPPA U, SIMONA C, SCHAPPI P, GRAF H, ESPELAND M. Episodic probing attachment loss in humans: histologic associations. J Periodontol 1990: 61: 420- 426.
116- ZAMBON J., HARASZTHY V. I. The laboratory diagnosis of periodontal infections. Periodontol. 2000,Vol.7, 1995, 69-82.
117- ZADEH H.H., NICHOLS F.C., MIYASAKI T. The role of the cell-mediated immune response to Actinobacillus actinomycetemcomitans and Porphyromonas gingivalis in periodontitis. Periodontol. 2000, Vol. 20, 1999 ; 239-288.
118- Expertise collective INSERM; Les Editions INSERM, 1999. Maladies parodontales. Thérapeutiques
   et Prevention.

## Revendications

1. Procédé de détection de la maladie parodontale utilisant des tests bactériens et génétiques dans lequel on détecte des variations spécifiques des gènes codants pour IL-1α⁻⁸⁸⁹ et IL-1β⁺³⁹⁵³

2. Procédé de détection de la maladie parodontale utilisant des tests bactériens et génétiques dans lequel on mesure l'allèle 2 de l'IL-1α⁻⁸⁸⁹ et/ou l'allèle 2 de l'IL-1β⁺³⁹⁵³

3. Procédé de détection de la maladie parodontale utilisant des tests bactériens et génétiques dans lequel l'allèle 2 de l'IL-1β⁺³⁹⁵³ interagit indépendamment ou avec un ou plusieurs gènes impliqués dans le développement des formes les plus sévères de maladie parodontale.

4. Procédé de détection de la maladie parodontale utilisant des tests bactériens et génétiques dans lequel le test génétique s'appuie sur le polymorphisme génétique qui caractérise les variations de réponse de l'hôte à l'activité microbienne, permettant d'évaluer la prédisposition d'un patient à développer une maladie parodontale à tendance sévère et/ou très évolutive, et ce même si les symptômes ne sont pas encore observables.

5. Procédé de détection de la maladie parodontale utilisant des tests bactériens et génétiques dans lequel le test génétique est un test génétique d'évaluation de la prédisposition des sujets testés au développement d'une maladie parodontale à expression inflammatoire.

6. Procédé de détection de la maladie parodontale utilisant des tests bactériens et génétiques dans lequel l'on combine les test de présence de bactéries en milieu buccal et les test génétiques pour évaluer la susceptibilité d'un patient à une maladie parodontale.

7. Procédé de détection tel que le test génétique est le test PST+ (Periodontal Susceptibility Test) qui détecte des variations spécifiques des gènes codant pour l'interleukine II-1α et II-1β.
